# EUROPEAN PATENT SPECIFICATION

(11) **EP 1 262 177 B1**
(45) Date of publication and mention of the grant of the patent: **23.08.2006**
(21) Application number: 02253607.2
(22) Date of filing: 22.05.2002
(51) Int. Cl.: A61K 31/53, A61P 17/14, A61K 8/49, A61Q 7/00

(54) **Medical use of thyromimetic compounds to treat hair loss and compositions**
Medizinische Verwendung thyromimetischer Verbindungen gegen Haarausfall und Zusammensetzungen
Utilisation médicale des composés thyromimétiques pour traiter la chute des cheveux et compositions

(30) Priority: 31.05.2001 US 294962 P
(43) Date of publication of application: 04.12.2002
(73) Proprietor: Pfizer Products Inc., Groton, Connecticut 06340 (US)
(72) Inventor: Chian, Yuan-Ching Phoebe c/o Pfizer Global R & D, Groton, Connecticut 06340 (US); Cornelius, Peter c/o Pfizer Global R & D, Groton, Connecticut 06340 (US); Doherty, Niall Stephen c/o Pfizer Global R & D, Groton, Connecticut 06340 (US); Dow, Robert Lee c/o Pfizer Global R & D, Groton, Connecticut 06340 (US)
(74) Representative: Rutt, Jason Edward

(56) References cited:
- EP-A- 1 088 819
- WO-A-00/51971
- WO-A-00/58279
- WO-A-00/72812
- WO-A-00/72920
- WO-A-00/73265
- WO-A-00/73292
- WO-A-01/70687
- WO-A-02/32408

## Description

### FIELD OF THE INVENTION

The present invention provides methods and compositions for treating hair loss, including arresting and/or reversing hair loss and promoting hair growth, in mammals, such as humans, companion animals and livestock, using certain thyromimetic compounds, as described below.

### BACKGROUND OF THE INVENTION

Hair loss is a common problem, which occurs, for example, through natural processes or is often chemically promoted through the use of certain therapeutic drugs designed to alleviate conditions, such as cancer. Often such hair loss is accompanied by lack of hair regrowth, which causes partial or full baldness.

As is well known in the art, hair growth occurs by a cycle of activity, which involves alternating periods of growth and rest. This cycle is often divided into three main stages, which are known as anagen, catagen and telogen. Anagen is the growth phase of the cycle and may be characterized by penetration of the hair follicle deep into the dermis with rapid proliferation of cells, which are differentiating to form hair. The next phase is catagen, which is a transitional stage marked by the cessation of cell division, and during which the hair follicle regresses through the dermis and hair growth is ceased. The next phase, telogen, is often characterized as the resting stage during which the regressed follicle contains a germ with tightly packed dermal papilla cells. At telogen, the initiation of a new anagen phase is caused by rapid cell proliferation in the germ, expansion of the dermal papilla, and elaboration of basement membrane components.

There have been many attempts in the literature to prevent the loss of hair or to invoke the regrowth of hair by, for example, the promotion or prolongation of anagen. Currently, there are two drugs approved by the United States Food and Drug Administration for the treatment of male pattern baldness: topical minoxidil (marketed as ROGAINE® by Pharmacia) and oral finasteride (marketed as PROPECIA® by Merck & Co., Inc.). For several reasons, however, including safety concerns and/or limited efficacy, the search for efficacious hair growth inducers is ongoing.

Two naturally occurring thyroid hormones, namely, thyroxine or 3,5,3',5'-tetraiodo-L-thyronine (commonly referred to as "T₄"), and thyronine or 3,5,3'-triiodo-L-thyronine (commonly referred to as "T₃"), are shown below: 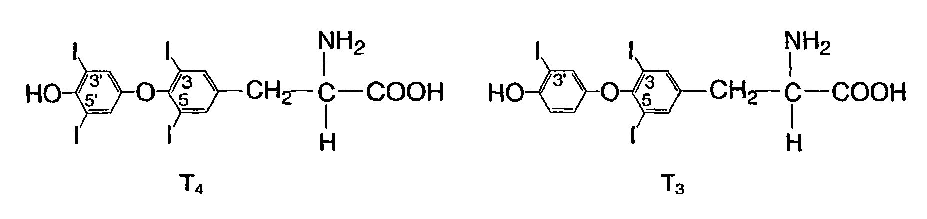 T₃ is the more biologically active of the two and, as will be appreciated from the structural formulae provided above, differs from T₄ by the absence of the 5' iodine. T₃ may be produced directly from the thyroid gland or, in peripheral tissues, by the removal of the 5' iodine by deiodinase enzymes. Thyromimetic analogs are often designed to be structurally similar to T₃. In addition, naturally occurring metabolites of T₃ are known.

Interestingly, it is known that the thyroid hormone, thyroxine ("T₄"), converts to triiodo-thyronine ("T₃") in human skin by deiodinase I, a selenoprotein. Selenium deficiency causes a decrease in T₃ levels due to a decrease in deiodinase I activity; this reduction in T₃ levels is strongly associated with hair loss. Consistent with this observation, hair growth is a reported side effect of administration of T₄. See, for example, Berman, "Periperal Effects of L-Thyroxine on Hair Growth and Coloration in Cattle," *Journal of Endocrinology,* Vol. 20, pp. 282-292 (1960); and Gunaratnam, "The Effects of Thyroxine on Hair Growth in the Dog," *J. Small Anim. Pract.,* Vol. 27, pp. 17-29 (1986). Furthermore, T₃ and T₄ have been the subject of several patent publications relating to treatment of hair loss. See, e.g., German patent 1,617,477; British patent 2,138,286; and WO 96/25943.

Thus, it is known that thyroid hormone can exert positive effects on hair growth; however, administration of T₃ and/or T₄ to treat hair loss is not practicable because these thyroid hormones are known to cause adverse side effects, such as inducing significant cardiotoxicity or adversely affecting bone mineral density and lean body mass. See, e.g., U.S. Patent No. 5,284,971; and U.S. Patent No. 5,061,798.

According to the present invention, it has been found that administration of certain thyromimetic compounds, as described below, which activate thyroid hormone receptors in certain tissues, including those in the hair follicle which control growth and hair production, but spare other tissues, such as the heart, could be used to increase hair growth in patients suffering from hair loss, or may be used to prevent or delay hair loss in patients just beginning to lose their hair.

Published International patent application WO 00/72810 discloses methods of treating hair loss using certain sulfonyl thyromimetic compounds. Published International patent application WO 00/72811 discloses methods of treating hair loss using certain compounds, such as substituted phenoxy-benzoic acid compounds, described therein. Published International patent application WO 00/72812 discloses methods of treating hair loss using certain diphenylether derivatives. Published International patent application WO 00/72813 discloses methods of treating hair loss using certain diphenylmethane derivatives. Published International patent application WO 00/72920 discloses certain substituted biaryl ether compounds and compositions for treating hair loss. Published International patent application WO 00/73292 discloses certain biaryl compounds and compositions for treating hair loss.

Commonly assigned, published International patent application WO 00/51971 and commonly assigned, published European patent application EP 1 033 364 disclose certain oxamic acids and derivatives thereof as thyroid receptor ligands. Commonly assigned, published European Patent Application EP 1 088 819 discloses certain 6-azauracil derivatives as thyroid receptor ligands. Commonly assigned, published European Patent Application EP 1 127 882 discloses certain tetrazole compounds as thyroid receptor ligands. Commonly assigned, published European Patent Application EP 1 148 054 discloses certain thiazolidinedione, oxadiazolidinedione and triazolone compounds, which are thyroid receptor ligands.

The following are recent articles on thyroid hormone receptors (TRs): M.K. Ahsan et al., J. Med. Invest. 44: 179-184, 1998, studied the immunohistochemical localization of thyroid hormone receptors (TRs) in human scalp skin and concluded that the results demonstrated the presence of thyroid hormone nuclear receptors in human hair follicles. N. Billoni et al., *British Journal of Dermatology* 2000: 142: 645-652, established that TRβ1 was the predominant form of TR expressed in the human hair follicle. C.C. Thompson and M.C. Bottcher, *Proc. Natl. Acad. Sci. USA,* Vol. 94, pp. 8527-8532, August 1997, found that the product of a thyroid hormone-responsive gene, the lack of which confers a hairless phenotype, interacts with thyroid hormone receptors. A.G. Messenger, *British Journal of Dermatology,* **142**, 631-635, 2000, discussed the relationship between thyroid hormone and hair growth.

J.D. Safer, L.M. Fraser, M. Hoa and M.F. Holick, "Intraperitoneal and Topical Triiodothyronine Have Opposing Effects on Mouse Skin," Abstract P1-530 for The Endocrine Society 83^{rd} Annual Meeting, which is scheduled to take place on June 20-23, 2001, in Denver, Colo.), discusses the differential effects of triiodothyronine on skin of mouse, depending on route of administration.

J. D. Safer, et al., Thyroid 11 (8): 717-24 (Aug. 2001), found topical triiodothyronine stimulates epidermal proloferation, dermal thickening and hair growth in mice and rats.

V. L. Malloy, et al., Abstract titled "Effect of Topically Applied Thyroid Hormone on Androgen Dependent Models of the Pilo-Sebaceous Apparatus," Clinical Research, Vol. 36, No. 5, page 814A (1998), observed an inhibitory effect on testosterone induced alopecia after treatment with topical T₃ in the AGA mouse, a model for androgen dependent hair loss.

### SUMMARY OF THE INVENTION

The present invention relates to methods for treating hair loss in mammals comprising administering certain compounds, as described below.

The present invention also relates to the use of certain compounds, as described below, for the manufacture or preparation of a medicament for the treatment of hair loss in mammals.

More particularly, the present invention provides such methods wherein the compounds are cardiac-sparing.

More particularly, the present invention provides such methods wherein the treatment is the arresting or reversing of hair loss.

More particularly, the present invention provides such methods wherein the treatment is the promotion of hair growth.

More particularly, the present invention provides such methods wherein the treatment is the acceleration of hair regrowth following chemotherapy-induced hair loss.

More particularly, the present invention provides such methods wherein the mammal is a human being.

More particularly, the present invention provides such methods wherein the compounds are administered topically.

More particularly, the present invention provides such methods which further comprise the administration of an effective amount of an agent for treating hair loss, e.g., finasteride, minoxidil or cyproterone acetate.

In addition, the present invention provides topical compositions for promoting hair growth which comprise an effective amount of certain compounds, as described below, and pharmaceutically acceptable carriers.

More particularly, the present invention provides such compositions wherein the topical composition is in the form of a lotion, cream, ointment, shampoo, paste, gel, spray, aerosol or kit. The present invention also provides such compositions which further comprise an effective amount of an agent for treating hair loss, e.g., finasteride, minoxidil or cyproterone acetate.

In addition, the present invention provides kits for treating hair loss in a mammal, the kit comprising:
a) a first pharmaceutical composition comprising a compound as described below;
b) a second pharmaceutical composition comprising an additional compound useful for treating hair loss; and
c) a container.

More particularly, the present invention provides such kits wherein the additional compound is finasteride, minoxidil or cyproterone acetate

### DETAILED DESCRIPTION OF THE INVENTION

The present invention relates to methods for treating hair loss in mammals, including arresting and/or reversing hair loss and promoting hair growth, comprising administering certain thyromimetic compounds, as described below.

Preferred mammals include humans, companion animals such as dogs, cats and horses, and livestock such as cattle, swine and sheep. Particularly preferred mammals include humans.

Preferably, in the methods of the present invention, the compounds are administered topically.

The preferred compounds useful in the methods of the present invention are cardiac-sparing. The term "cardiac-sparing" as used herein means that, at the dosages required for hair growth, the compounds useful in the methods of the present invention do not produce any observable cardiotoxicity in the mammal being treated.

All percentages, ratios and proportions used herein are by weight unless otherwise specified.

As used herein, "effective amount of a compound" means an amount that is effective to exhibit biological activity, preferably wherein the biological activity is arresting and/or reversing hair loss or promoting hair growth, at the site(s) of activity in a mammalian subject, without undue adverse side effects (such as undue toxicity, irritation or allergic response), commensurate with a reasonable benefit/risk ratio when used in the manner of the present invention.

The phrase "compound(s) useful in the methods of the present invention," shall at all times be understood to include all active forms of such compounds, including, for example, the free form thereof, e.g., the free acid or base form, and also, polymorphs, hydrates, solvates, tautomers, stereoisomers, e.g., diastereomers and enantiomers, and all pharmaceutically acceptable salts as described above, unless specifically stated otherwise. It will also be appreciated that suitable active metabolites of such compounds, in any suitable form, are also included herein.

The present invention includes the use of the thyromimetic compounds of the following formula, also described in commonly assigned, published European patent application EP 1 088 819: 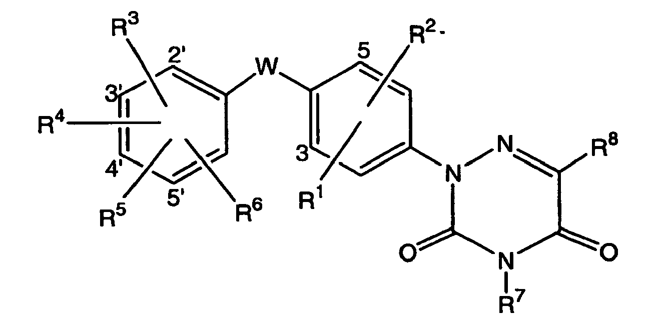
an isomer thereof, or a pharmaceutically acceptable salt of said compound or isomer; wherein W is (a) -O-, (b) -S(O)ₘ-, (c) -NR³⁰-, (d) -C(O)-, (e) -HC=CH-, (f) -CH₂-, (g) -CHF-, (h) -CF₂- or (i) -CH(OH)-;
R¹ and R² are independently (a) hydrogen, (b) halogen, (c) -(C₁-C₆)alkyl, (d) -CN, (e) -OR¹² or (f) -trifluoromethyl;
R³ is (a) hydrogen, (b) halogen, (c) -(C₁-C₆)alkyl optionally substituted with one to three substituents independently selected from the group consisting of halogen, -OCF₃ and -CF₃, (d) -CN, (e) -OR¹², (f) -trifluoromethyl, (g) -NO₂, (h) -SO₂₋R¹³, (i) -C(O)₂R⁹, (j) -C(O)NR¹⁹R²⁰, (k) -C(O)R¹⁶, (I) -NR²¹C(O)-NR²¹R²², (m) -NR¹⁹⁻C(O)R²⁰ or (n) -NR¹⁷R¹⁸;
R⁴ is (a) -C(R¹⁴)(R¹⁵)(R¹⁶), (b) -(C₀-C₃)alkyl-NR¹⁷R¹⁸, (c) -C(O)NR¹⁹R²⁰, (d) -NR¹⁹-C(O)-R²⁰, (e) -(C₀-C₃)alkyl-NR²¹-C(O)-NR²¹R²², (f) -S(O)ₘ-R²², (g)-S(O)₂₋NR²¹R²², (h) -NR²¹-S(O)₂-R²², (i) -aryl, (j) -het, (k) -OR³³ or (I) halogen; provided that in substituents (f) and (h), R²² is other than -OR³⁴; and provided that when substituent (b) is -(C₀)alkyl-NR¹⁷R¹⁸, R¹⁸ is other than -C(O)-R²⁸ or -S(O)₂-R²⁹;
or R³ and R⁴ may be taken together to form a carbocyclic ring of Formula -(CH₂)_{b}- or a heterocyclic ring selected from the group consisting of -O-(CH₂)_{c}- and -(CH₂)ⱼ-Q-(CH₂)ₖ- wherein Q is O, S or NR²⁵; wherein said carbocyclic ring is optionally substituted with one or more substituents independently selected from Group V; and wherein said heterocyclic ring is optionally substituted with one or more substituents independently selected from Group Z;
R⁵ is -OR²³;
or R⁴ and R⁵ may be taken together to form a heterocyclic ring selected from the group consisting of -CR³¹=CR³²-NH-, -N=CR³¹-NH-, -CR³¹=CR³²-O- and -CR³¹=CR³²-S-;
R⁶ is (a) hydrogen, (b) halogen, (c) -(C₁-C₆)alkyl optionally substituted with one to three substituents independently selected from the group consisting of halogen, -OCF₃ and -CF₃, (d) -CN, (e) -OR¹², (f) -trifluoromethyl, (g) -NO₂, (h) -SO₂₋R¹³, (i) -C(O)₂R⁹, (j) -C(O)NR¹⁹R²⁰, (k) -C(O)R¹⁶, (l) -NR²¹C(O)NR²¹R²², (m) -NR¹⁹⁻C(O)R²⁰ or (n) -NR¹⁷R¹⁸;
R⁷ is (a) hydrogen, (b) -(C₁-C₄)alkyl wherein each carbon atom is optionally substituted with 1 to 3 halo atoms or (c) -(CH₂)ₙCOOR⁹;
R⁸ is (a) hydrogen, (b) -(C₁-C₆)alkyl, (c) -C(O)-OR⁹, (d) -C(O)NR¹⁰R¹¹ or (e) -CN; provided that in substituent (c), R⁹ is other than methyl or ethyl; and provided that in substitutent (d), R¹⁰ and R¹¹ are not both hydrogen;
R⁹ is (a) -(C₁-C₁₂)alkyl optionally substituted with one or more substitutents independently selected from Group V, (b) -(C₂-C₁₂)alkenyl optionally substituted with phenyl, (c) -(C₂-C₁₂)dialkenyl, (d) -(C₃-C₁₀)cycloalkyl, (e) -aryl or (f) -het;
R¹⁰ and R¹¹ are independently (a) hydrogen, (b) -(C₁-C₁₂)alkyl optionally substituted with one or more substituents independently selected from Group V, (c) -(C₃-C₁₀)cycloalkyl optionally substituted with one or more substituents independently selected from Group V, (d) -(C₂-C₁₂)alkenyl or (e) -het;
or R¹⁰ and R¹¹ for any occurrence may be taken together with the nitrogen atom to which are they attached to form het;
R¹² is (a) hydrogen or (b) -(C₁-C₆)alkyl wherein each carbon atom is optionally substituted with 1 to 3 fluoro atoms;
R¹³ is (a) -(C₁-C₁₂)alkyl optionally substituted with one or more substituents independently selected from Group V, (b) -(C₂-C₁₂)alkenyl, (c) -(C₃-C₁₀)cycloalkyl, (d) -NR¹⁷R¹⁸, (e) -aryl or (f) -het;
R¹⁴ is (a) hydrogen, (b) -(C₁-C₆)alkyl or (c)-O-R³⁴;
R¹⁵ is (a) hydrogen or (b) -(C₁-C₆)alkyl;
or R¹⁴ and R¹⁵ are taken together with the carbon atom to which they are attached to form a carbonyl group;
R¹⁶ is (a) hydrogen, (b) -(C₁-C₆)alkyl wherein each carbon atom is optionally substituted with 1 to 3 fluoro atoms, (c) -(C₀-C₆)alkyl-(C₃-C₁₀)cycloalkyl, (d) -(C₀₋C₆)alkyl-aryl or (e) -(C₀-C₆)alkyl-het;
R¹⁷ is (a) hydrogen, (b) -(C₁-C₁₂)alkyl optionally substituted with one or more substituents independently selected from Group V, (c) -aryl, (d) -het, (e) -OR³⁴ or (f) -(C₃-C₁₀)cycloalkyl;
R¹⁸ is (a) hydrogen, (b) -(C₁-C₁₂)alkyl optionally substituted with one or more substituents independently selected from Group V, (c) -aryl, (d) -het, (e) -C(O)-R²⁸, (f) -S(O)₂-R²⁹, (g) -OR³⁴ or (h) -(C₃-C₁₀)cycloalkyl;
or R¹⁷ and R¹⁸ for any occurrence are taken together with the nitrogen atom to which they are attached to form het;
R¹⁹ and R²⁰ for each occurrence are independently
   (a) hydrogen, (b) -(C₁-C₁₂)alkyl optionally substituted with one or more substituents independently selected from Group V, (c) -(C₀-C₆)alkyl-aryl,
   (d) -(C₀-C₆)alkyl-het, (e) -C(O)-NR²⁶R²⁷, (f) -C(O)-R²⁸, (g) -S(O)₂-R²⁹ , (h) -OR³⁴ or (i) -(C₃-C₁₀)cycloalkyl;
or R¹⁹ and R²⁰ for any occurrence are taken together with the nitrogen atom to which they are attached to form het;
R²¹ and R²² for each occurrence are independently
   (a) hydrogen, (b) -(C₁-C₁₂)alkyl optionally substituted with one to three substituents independently selected from Group V, (c)-aryl, (d) -het, (e) -(C₃-C₁₀)cycloalkyl or (f) -OR³⁴;
or R²¹ and R²² are taken together with the nitrogen atom to which they are attached to form het;
R²³ is (a) hydrogen, (b) -(C₁-C₄)alkyl optionally substituted with one or more substituents independently selected from Group V or (c) -C(O)-R²⁴;
R²⁴ is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R²⁵ for each occurrence is independently (a) hydrogen, (b) -(C₁-C₆)alkyl,
   (c) -COR²⁹ or (d) -SO₂R²⁹;
R²⁶ and R²⁷ for each occurrence are independently (a) hydrogen,
   (b) -(C₁-C₆)alkyl, (c) -(C₃-C₁₀)cycloalkyl, (d) -(C₀-C₆)alkyl-aryl, or (e) -(C₀-C₆)alkyl-het,
R²⁸ is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R²⁹ is (a) -(C₁-C₁₂)alkyl optionally substituted with one or more substituents independently selected from Group V, (b) -(C₂-C₁₂)alkenyl,
   (c) -(C₃-C₁₀)cycloalkyl, (d) -aryl or (e) -het;
R³⁰ is (a) hydrogen, (b) -(C₁-C₁₂)alkyl optionally substituted with one or more substituents independently selected from Group V, (c) -(C₁-C₁₂)alkenyl,
   (d) -(C₃-C₁₀)cycloalkyl, (e) -C(O)-R³¹ or (f) -S(O)ₘ-R³²;
R³¹ is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl, (f) -het or (g) -OR³⁴;
R³² is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R³³ is (a) -(C₀-C₆)alkyl-aryl, (b) -(C₀-C₆)alkyl-het, (c) -(C₇-C₁₂)alkyl optionally substituted with one or more substituents independently selected from Group V, (d) -(C₁-C₆)alkyl wherein at least one carbon atom is substituted with 1 to 3 fluoro atoms, (e) -(C₂-C₁₂)alkenyl or (f) -(C₃-C₁₀)cycloalkyl;
R³⁴ is (a) -aryl, (b) -het , (c) -(C₁-C₁₂)alkyl optionally substituted with one or more substituents independently selected from Group V, (d) -(C₂-C₁₂)alkenyl or (e) -(C₃-C₁₀)cycloalkyl;
-(C₃-C₁₀)cycloalkyl for each occurrence is a fully or partially saturated mono-, bi- or tricyclic ring containing three to ten carbon atoms; wherein in the bicyclic ring, a monocyclic cycloalkyl ring is spiro fused to another cycloalkyl ring or is fused via two carbon atoms to a benzene ring or another cycloalkyl ring; and wherein in the tricyclic ring, a bicyclic ring is spiro fused to a cycloalkyl ring or is fused via two atoms to a benzene ring or another cycloalkyl ring;
said -(C₃-C₁₀)cycloalkyl optionally contains one to three bridging atoms independently selected from carbon, oxygen, sulfur and nitrogen; said bridging atoms are attached to two carbon atoms in the ring; and said bridging atoms are optionally substituted with one to three groups independently selected from -(C₁₋C₆)alkyl and hydroxy;
said cycloalkyl ring is optionally substituted on one ring if the moiety is monocyclic, on one or both rings if the moiety is bicyclic, or on one, two or three rings if the moiety is tricyclic, with one or more substitutents independently selected from Group V;
Group V is (a) -(C₁-C₆)alkyl optionally substituted with one or two hydroxy, (b) -(C₂-C₅)alkynyl, (c) -halogen, (d) -NR³⁵R³⁶, (e) -NO₂, (f) -OCF₃, (g) -OR³⁷, (h) -SR³⁷, (i) -oxo, (j) -trifluoromethyl, (k) -CN, (l) -C(O)NR³⁵-OH, (m) -COOR³⁵, (n) -O-C(O)-(C₁-C₆)alkyl, (O) -(C₃-C₁₀)cycloalkyl optionally substituted with CN, (p) -(C₀₋C₆)alkyl-aryl, (q) -(C₀-C₆)alkyl-het, (r) -C(O)-(C₁-C₆)alkyl or (s) -C(O)-aryl;
R³⁵ and R³⁶ for each occurrence are independently (a) hydrogen, (b) -(C₁₋C₆)alkyl or (c) -(C₀-C₆)alkyl-aryl;
R³⁷ is (a) hydrogen, (b) -(C₁-C₆)alkyl optionally substituted with one or more halo, hydroxy or methoxy, (c) -(C₀-C₆)alkyl-aryl or (d) -(C₀-C₆)alkyl-het;
aryl is (a) phenyl optionally substituted with one or more substituents independently selected from Group Z; (b) naphthyl optionally substituted with one or more substituents independently selected from Group Z or (c) biphenyl optionally substituted with one or more substituents independently selected from Group Z;
het for each occurrence is a 4-, 5-, 6-, 7- and 8-membered fully saturated, partially saturated or fully unsaturated mono-, bi- or tricyclic heterocyclic ring containing from one to four heteroatoms independently selected from the group consisting of oxygen, sulfur and nitrogen; wherein in the bicyclic ring, a monocyclic heterocyclic ring is spiro fused to a -(C₃-C₈)cycloalkyl ring or to another heterocyclic ring which is fully or partially saturated; or is fused via two atoms to a benzene ring, a -(C₃-C₈)cycloalkyl ring or another heterocyclic ring; and wherein in the tricyclic ring, a bicyclic ring is spiro fused to a -(C₃-C₈)cycloalkyl ring or to another heterocyclic ring which is fully or partially saturated; or is fused via two atoms to a benzene ring, a (C₃-C₆)cycloalkyl ring, or another heterocyclic ring;
said het optionally contains one to three bridging atoms independently selected from oxygen, sulfur and nitrogen; said bridging atoms are attached to two other atoms in the ring; and said bridging atoms are optionally substituted with one to three groups independently selected from -(C₁-C₆)alkyl and hydroxy;
said het optionally has one or two oxo groups substituted on carbon or one or two oxo groups substituted on sulfur;
said het is optionally substituted on carbon or nitrogen, on one ring if the moiety is monocyclic, on one or both rings if the moiety is bicyclic, or on one, two or three rings if the moiety is tricyclic, with one or more substituents independently selected from Group Z;
Group Z for each occurrence is independently (a) hydrogen, (b) halogen, (c) trifluoromethyl, (d) hydroxy, (e) -OCF₃, (f) -CN, (g) -NO₂, (h) -(C₁-C₆)alkyl optionally substituted with one or more substituents independently selected from the group consisting of hydroxy, halogen, -OCF₃ and -CF₃, (i) -(C₂-C₆)alkenyl optionally substituted with phenyl, (j) -(C₂-C₅)alkynyl, (k) -(C₁-C₆)alkoxy, (I) -(C₀₋C₆)alkyl-phenyl optionally substituted with one or more substituents independently selected from the group consisting of halogen, -OCF₃, -CF₃, -(C₁-C₄)alkyl, -(C₁₋C₄)alkoxy and -C(O)CH₃, (m) -(C₀-C₆)alkyl-naphthyl optionally substituted with one or more substituents independently selected from the group consisting of halogen, -OCF₃, -CF₃, -(C₁-C₄)alkyl, -(C₁-C₄)alkoxy and -C(O)CH₃, (n) -C(O)₂R³⁵, (o) -(C₀₋C₆)alkyl-C(O)NR³⁵R³⁶, (p) -(C₀-C₆)alkyl-C(O)R³⁸, (q) -NR³⁵R³⁶, (r) -NR³⁵⁻C(O)NR³⁵R³⁶, (s) -NR³⁵-C(O)R³⁶, (t) -OR³⁷, (u) -SR³⁷, (v) -(C₃-C₁₀)cycloalkyl, (w) -(C₀-C₆)alkyl-pyridinyl optionally substituted with one or more -(C₁-C₆)alkyl which is optionally substituted with one or more substituents independently selected from the group consisting of hydroxy and halo, (x) -(C₀-C₆)alkyl-piperidinyl optionally substituted with one or more -(C₁-C₆)alkyl which is optionally substituted with one or more substituents independently selected from hydroxy and halo, (y) -SO₂-R³⁷, (z) -SO₂-NR³⁵R³⁶ or (a1) -S-phenyl-CH₂OH
R³⁸ is (a) -(C₁-C₆)alkyl, (b) -(C₀-C₆)alkyl-phenyl, (c) -(C₀-C₆)alkyl-phenanthrenyl optionally substituted with one to three CF₃, (d) -(C₀-C₆)alkyl-pyrrolidinyl or (e) -(C₀-C₆)alkyl-morpholinyl;
or any two Z Groups for any occurrence in the same variable may be taken together to form (a) a carbocyclic ring of the formula -(CH₂)ₑ- or (b) a heterocyclic ring selected from the group consisting of -O(CH₂)_{f}O-, -(CH₂)_{g}NH- and -CH=CHNH-;
m is 0, 1 or 2;
n is 0, 1, 2 or 3;
b is 3, 4, 5, 6 or 7;
c, f, g, j and k are each independently 2, 3, 4, 5 or 6; and
e is 3, 4, 5, 6 or 7;
provided that in a compound of the above formula: 1) the substituent -C(R¹⁴)(R¹⁵)(R¹⁶) in R⁴ is other than (C₁-C₄)alkyl; and 2) R⁴ is halo only when R⁸ is -C(O)-OR⁹ or -C(O)NR¹⁰R¹¹.

More particularly, the following compounds are useful in the methods of the present invention:
8-[[5-[2,6-dichloro-4-(4,5-dihydro-3,5-dioxo-1,2,4-triazine-2(3H)-yl)phenoxy]-2-hydroxyphenyl]sulfonyl]-spiro[8-azabicyclo[3.2.1]octane-3,2'-(3'H)-dihydro-furan];
2-{3,5-dichloro-4-[3-(3,3-dimethyl-piperidine-1-sulfonyl)-4-hydroxy-phenoxy]-phenyl}-2H-[1,2,4]triazine-3,5-dione;
2-{3,5-dichloro-4-[4-hydroxy-3-(3-methyl-3-phenyl-piperidine-1-sulfonyl)-phenoxy]-phenyl}-2H-[1,2,4]triazine-3,5-dione;
N-cyclohexyl-5-[2,6-dichloro-4-(3,5-dioxo-4,5-dihydro-3H-[1,2,4]triazin-2-yl)-phenoxy]-2-hydroxy-benzenesulfonamide;
N-bicyclo[2.2.1]hept-2-yl-5-[2,6-dichloro-4-(3,5-dioxo-4, 5-dihydro-3H-[1,2,4]triazin-2-yl)-phenoxy]-2-hydroxy-benzamide;
2-{3,5-dichloro-4-[3-(3,3-dimethyl-piperidine-1-carbonyl)-4-hydroxy-phenoxy]-phenyl}-2H-[1,2,4]triazine-3,5-dione;
N-bicyclo[2.2.1]hept-2-yl-5-[2,6-dichloro-4-(3,5-dioxo-4,5-dihydro-3H-[1,2,4]triazin-2-yl)-phenoxy]-2-hydroxy-benzamide;
2-{3,5-dichloro-4-[4-hydroxy-3-(3-methyl-3-phenyl-piperidine-1-carbonyl)-phenoxy]-phenyl}-2H-[1,2,4]triazine-3,5-dione;
5-[2,6-dichloro-4-(3,5-dioxo-4,5-dihydro-3H-[1,2,4]triazin-2-yl)-phenoxy]-N-(6,6-dimethyl-bicyclo[3.1.1]hept-2-yl)-2-hydroxy-benzamide;
2-{3,5-dichloro-4-[3-(3,5-dimethyl-piperidine-1-carbonyl)-4-hydroxy-phenoxy]-phenyl}-2H-[1,2,4]triazine-3,5-dione;
2-{3,5-dichloro-4-[4-hydroxy-3-(piperidine-1-carbonyl)-phenoxy]-phenyl}-2H-[1,2,4]triazine-3,5-dione;
N-cyclohexyl-5-[2,6-dichloro-4-(3,5-dioxo-4,5-dihydro-3H-[1,2,4]triazin-2-yl)-phenoxy]-2-hydroxy-benzamide;
2-{3,5-dichloro-4-[3-(3,4-dihydro-1H-isoquinoline-2-carbonyl)-4-hydroxy-phenoxy]-phenyl}-2H-[1,2,4]triazine-3,5-dione;
2-{4-[3-(4-fluoro-benzyl)-4-hydroxy-phenoxy]-3,5-dimethyl-phenyl}-2H-[1,2,4]triazine-3,5-dione; and
2-{3,5-dichloro-4-[3-(4-fluoro-benzoyl)-4-hydroxy-phenoxy]-phenyl}-2H-[1,2,4]triazine-3,5-dione.

Methods for making the thyromimetic compounds described above are disclosed in the above cited patent applications.

The ability of a thyromimetic compound to bind thyroid hormone receptors may be demonstrated in standard assays known in the art, such as the Thyroid Hormone Receptor Binding Assay, described at page 53 of published European application EP 1 088 819. Preferably, the thyromimetic compounds useful in the methods of the present invention are TRβ1-selective in the Binding Assay and, therefore, are more selective for the predominant form of the receptor present in human hair follicles, as recently stated in the art. As such, these compounds are expected to have a preferential effect on hair growth relative to cardiac endpoints and other undesirable endpoints.

Also, as noted above, preferably, the compounds useful in the present invention are cardiac-sparing. Compounds may be tested for their cardiac-sparing properties using the following assay:

### Cardiotoxicity Assay

As is well known by those skilled in the art, thyroid hormones affect cardiac functioning, for example, by causing an increase in the heart rate as well as an increase in tissue mass, or hypertrophy. The ability of the compounds useful in the methods of the present invention to cause the thyroid hormone-like, cardiotoxic effects may be demonstrated according to the following protocol:

### A. Experimental Summary

This *in vivo* screen is designed to evaluate the cardiac effects of compounds that are thyromimetic. The cardiac endpoints that are measured are heart weight and heart mitochondrial alpha-glycerophosphate dehydrogenase ("mGPDH") activity. The protocol involves: (a) dosing rodents for about 6 days, (b) harvesting tissue and weighing it, (c) preparing a subcellular fraction of the tissue, enriched in mitochondria, and (d) subsequent assaying of enzyme activity thereby.

### B. Preparation of Animals

A compound useful in the methods of the present invention, and a vehicle, or T₃ sodium salt, is administered topically or orally as a single daily dose given between about 3 p.m. to about 6 p.m. for about 6 days.

Animals are sacrificed by decapitation, tissues are dissected and weighed (for example, heart), then placed into 10 ml of cold homogenization buffer (0.25 M sucrose, 25 mM HEPES, pH 7.4, 0.5 mM EDTA, 0.5 mM AEBSF, 1 *µ*g/ml leupeptin), stored on ice. Tissue homogenates are prepared using a Polytron® homogenizer (Kinematica AG, Switzerland), and then centrifuged at 15,000 x g (11000 rpm, 10 minutes, at 4°C using a Sorvall® SM-24 rotor (Dupont)), after which the supernatant is discarded. The pellet is resuspended in homogenization buffer containing 0.1 % Triton-x 100 (6 mL), followed by sonication for 30 seconds (Branson Sonifier (Branson, Eagle Rd., Danbury, CT 06810), setting #2). Aliquots (1 ml in duplicate) are stored at -80°C, and 20*µ*l is placed in a separate tube for determination of the total protein concentration in the homogenate, using the BCA Protein Assay Kit (Pierce, 3747 No. Meridian Rd., Rockford, III 61105).

The mGPDH enzyme assay is carried out by incubating a sample of the tissue homogenate (containing a range of protein amounts, from 10 - 100 ug) prepared as described above, in a buffer of the following composition: 225 mM mannitol, 75 mM sucrose, 20 mM HEPES, pH 7.4, 50 mM KH₂PO₄, 1 mM KCN, 0.0025 mM rotenone, 0.025 mM menadione, 0.06 mM 2,6 dichlororindophenol. The assay is carried out at 37°C in a Molecular Devices SpectraMax 96-well plate spectrophotometer (1311 Orleans Drive, Sunnyvale, CA 94089), by addition of substrate (α-glycerophosphate) to a final concentration of 50 mM. The decline in absorbance at 600 nm wavelength is measured frequently over the next 30-60 minutes. The enzyme activity is calculated from the change in absorbance at 600 nM versus time. Finally, the change in absorbance at 600 nm per unit of time is divided by the amount of protein used in the assay. By comparing the mGPDH enzyme activity in cardiac tissue from control animals dosed with a vehicle solution to the mGPDH enzyme activity in cardiac tissue from animals treated with a thyromimetic compound, the cardiac effect of the thyromimetic compound can be assessed. Another effect of a thyromimetic compound on the heart is hypertrophy. Cardiac hypertrophy in response to a thyromimetic compound can be assessed by comparing the heart weight in control animals dosed with a vehicle solution to the heart weight in animals dosed with a thyromimetic compound.

### Telogen Conversion Assay

The Telogen Conversion Assay measures the potential of a compound (hereinafter referred to as the "test compound") to convert mice in the resting stage of the hair growth cycle ("telogen") to the growth stage of the hair growth cycle ("anagen"). Without intending to be limited by theory, there are three principal phases of the hair growth cycle: anagen, catagen and telogen. The telogen period in C3H/HeN mice lasts from approximately 40 days of age until about 75 days of age, when hair growth is synchronized. It is believed that after 75 days of age, hair growth is no longer synchronized. For example, about 40 day-old mice with dark fur (brown or black) may be used in hair growth experiments; melanogenesis occurs in these animals along with hair (fur) growth and the topical application of hair growth inducers may be evaluated in these animals.

The Telogen Conversion Assay described below is used to screen test compounds for potential hair growth by measuring melanogenesis:
Objectives: To evaluate and compare the effect of test compounds applied topically on C3H/HeN mice for hair growth.
Animals: Female C3H/HeN mice, six and a half weeks of age.

### Experimental Procedures:

Route and Duration of Treatment: Topical dosing with test compounds on mice is scheduled once or twice daily for five (5) consecutive days for one week to four weeks. Topical dosing begins on Day 0 by applying test compound or vehicle in volumes of 20 µl which keeps the solubilized test compound to a prescribed area of approximately one (1) square centimeter (1 cm²) in the center of the clipped lower back of each mouse.

Study Design: Only mice with pink skin or in the telogen phase of the hair growth cycle are selected for inclusion in the study. On Friday, before the experiment starts, the mice are weighed and then anesthetized with isoflurane in a mouse chamber. The hair over the lower dorsal area is clipped with a Wahl clipper using a #40 blade. Care is taken to avoid abrading the skin surface. On the Monday (Day 0) following hair clipping, the mice are again anesthetized with isoflurane and photographed with a digital camera. After digital photographs are taken, the mice receive 20µl of the respective test compounds applied with an automated pipette to the clipped area between the hind legs. The test compound for each treatment group is evenly spread with the pipette tip to an area approximately 1 square centimeter (1 cm²), and applied to the same site on subsequent dosing days. All topical treatments are administered as described in the Route and Duration of Treatment Section.

Observations of the test sites are made three (3) times a week on Monday, Wednesday, and Friday, with observations beginning immediately after the first week of treatment and looking for changes in skin pigment, hair growth, and signs of irritation such as scaling, peeling, scabbing and erythema. Digital photographs are taken on Days 0, 10, 15, 31 and 35 (end of the study) for the dosing groups. Body weights of mice are taken on the first and last days of observation for each test group.

The scoring system for hair growth is described below:
0 = No hair growth/ pigment change (pink skin);
1 = Gray/dark skin color (no visible hair growth);
2 = Sparse/diffuse terminal hair growth;
3 = dense, normal hair growth.
ph 2 = peripheral hair growth with a score of 2
ph 3 = peripheral hair growth with a score of 3
Idsh = long, diffuse/scattered hair growth in the dosing site
Scaling, peeling, scabbing, or erythema are scored as present (+) and absent (-). Any other local (e.g. test compound precipitation) or systemic abnormalities and aberrant hair growth are described on raw data scoring sheet.

Table 1 is a summary table of the results of the evaluation of five different test compounds in the Telogen Conversion Assay described above. Animals were observed and scored for hair growth using the scoring system for hair growth described above.

**Table 1.**

| Treatment Group | Experiment Number | LDO¹ | Hair Growth at LDO (% Animals)² | n³ | Hair Growth in 50% of Animals (Day)⁴ |
|---|---|---|---|---|---|
| PG:EtOH (30:70) 1wk | 1 | 30 | 22 | 9 | >30 |
| Cpd #1 (0.001 %) 1wk | 1 | 30 | 22 | 9 | >30 |
| Cpd #1 (0.01 %) 1wk | 1 | 30 | 75 | 8 | 14 |
| Cpd #1 (0.1 %) 1wk | 1 | 30 | 100 | 9 | 7 |
| Cpd #1 (0.3%) 1wk | 1 | 30 | 100 | 9 | 9 |
| | | | | | |
| PG:EtOH (30:70) 2wks | 2 | 63 | 0 | 9 | 56 |
| Cpd #1 (0.001 %) 1wk | 2 | 35 | 50 | 10 | 18 |
| Cpd #1 (0.01 %) 1wk | 2 | 35 | 100 | 10 | 14 |
| Cpd #1 (0.1%) 1wk | 2 | 35 | 100 | 10 | 7 |
| Cpd #1 (0.3%) 1wk | 2 | 35 | 100 | 10 | 9 |
| | | | | | |
| PG:EtOH (30:70) 2wks | 3 | 35 | 30 | 10 | >35 |
| Cpd #2 (0.1%) 2wks | 3 | 35 | 100 | 10 | 11 |
| Cpd #4 (0.1 %) 2wks | 3 | 35 | 100 | 10 | 9 |
| Cpd #5 (0.1 %) 2wks | 3 | 35 | 100 | 10 | 9 |
| Cpd #1 (0.1 %) 2wks | 3 | 35 | 100 | 10 | 7 |
| Cpd #1 (0.1%) 1wk | 3 | 35 | 100 | 10 | 7 |
| PG:EtOH (30:70) 1wk | 3 | 35 | 20 | 10 | >35 |

| | | | | | |
|---|---|---|---|---|---|
| ¹LDO is the last day of observation. | | | | | |
| ²Percentage (%) of study animals that had a hair growth score of 1 or greater on the last day of observation (LDO). | | | | | |
| ³n is the total number of animals in a group. | | | | | |
| ⁴Day of observation when 50% or greater of the study animals had a hair growth score of 1 or greater. | | | | | |

The following are the test compounds (Cpd#) for which results are summarized in the above Table 1:
Compound #1: N-Cyclohexyl-5-[2,6-dichloro-4-(3,5-dioxo-4,5-dihydro-3H-[1,2,4]triazin-2-yl)-phenoxy]-2-hydroxy-benzamide;
Compound #2: 2-{3,5-Dichloro-4-[3-(3,4-dihydro-1H-isoquinoline-2-carbonyl)-4-hydroxy-phenoxy]-phenyl}-2H-[1,2,4]triazine-3,5-dione;
Compound #4: 2-{4-[3-(4-Fluoro-benzyl)-4-hydroxy-phenoxy]-3,5-dimethylphenyl}-2H-[1,2,4]triazine-3,5-dione;
Compound #5: 2-{3,5-Dichloro-4-[3-(4-fluoro-benzoyl)-4-hydroxy-phenoxy]-phenyl}-2H-[1,2,4]triazine-3,5-dione.

There are known in the art other *in vitro* and *in vivo* assays models for understanding the biology of hair growth and for identifying the mechanisms that control hair growth and the hair growth cycle. These models are amenable to determining the effect of a compound useful in the methods of the present invention on hair growth.

For example, M.P. Philpott, D.A. Sanders and T. Kealey, *Dermatologic Clinics,* 14(4):595-607, October 1996, describes an *in vitro* culture of whole hair follicles for studying the biology of hair growth. M.P. Philpott and T. Kealey, *Journal of Investigative Dermatology,* 115(6):1152-5, December 2000, describes the rat vibrissa follicle which is an *in vitro* model system to investigate hair growth cycle control. M. Philpott, *Experimental Dermatology,* 8(4):317-9, August 1999, describes and references the current status and future development of *in vitro* models for the maintenance of isolated hair follicles which are useful for investigating the biology of hair growth. D. Van Neste, in H.I. Maibach (Ed.), "Dermatologic Research Techniques," pp. 37-49, CRC Press, Boca Raton (1996), reviews the techniques for the use of scalp grafts onto nude mice as a model for human hair growth. H. Uno, B. Schroder, T. Fors and O. Mori, *Journal of Cutaneous Aging & Cosmetic Dermatology,* Vol. 1, No. 3, 1990, pp 193-204 describes and references the stumptailed macaque, Sprague-Dawley rat and C3H mice as models for the study of hair growth *in vivo.*

The methods of the present invention are performed by administering to a mammal (preferably a human) a compound as described herein and preferably, a pharmaceutically acceptable or cosmetically acceptable carrier.

The compounds described herein may be used for the treatment of such conditions as treating hair loss in mammals, including arresting and/or reversing hair loss and promoting hair growth. Such conditions may manifest themselves in, for example, alopecia areata, including male pattern baldness and female pattern baldness. The compounds described herein may also be used to accelerate the regrowth of hair following chemotherapy-induced hair loss.

Preferably, in the methods of the present invention, the compounds are formulated into pharmaceutical or cosmetic compositions for use in treatment or prophylaxis of conditions, such as the foregoing. Standard pharmaceutical formulation techniques are used, such as those disclosed in Remington's Pharmaceutical Sciences, Mack Publishing Company, Easton, PA. (1990).

Typically, from about 0.01 mg to about 3000 mg, more preferably from about 0.05 mg to about 1000 mg, more preferably from about 0.10 mg to about 100 mg, of a compound as described herein is administered per day for systemic administration. For topical administration, typically, from about 0.0001% to about 10% (w/v), more preferably from about 0.0001 % to about 1 % (w/v), more preferably from about 0.0001 % to about 0.1% (w/v), of a compound as described herein is administered per day.

However, it is understood that daily administration of a compound as described herein can be adjusted depending on various factors. The specific dosage of the compound to be administered, as well as the duration of treatment, and whether the treatment is topical or systemic are interdependent. The dosage and treatment regimen will also depend upon such factors as the specific compound used, the treatment indication, the efficacy of the compound, the personal attributes of the subject (such as, for example, weight, age, sex and medical condition of the subject), compliance with the treatment regimen, and the presence and severity of any side effects of the treatment.

According to the present invention, the compounds as described herein are co-administered with a pharmaceutically acceptable or cosmetically acceptable carrier (herein collectively described as a "carrier"). The term "carrier," as used herein, means one or more compatible solid or liquid filler diluents, vehicles or encapsulating substances, which are suitable for administration to a mammal. The term "compatible," as used herein, means that the components of the composition are capable of being commingled with a compound as described herein, and with each other, in a manner such that there is no interaction which would substantially reduce the efficacy of the composition under ordinary use situations. Carriers must, of course, be of sufficiently high purity and sufficiently low toxicity to render them suitable for administration to the mammal (preferably the human being) being treated. The carrier can itself be inert or it can possess pharmaceutical and/or cosmetic benefits of its own.

The compounds useful in the methods of the present invention may be formulated in any of a variety of forms suitable, for example, for oral, topical or parenteral administration. Of these, topical administration is preferred.

Depending upon the particular route of administration desired, a variety of carriers well known in the art may be used. These include solid or liquid fillers, diluents, hydrotropes, surface active agents and encapsulating substances. Optional pharmaceutically active or cosmetically active materials may be included which do not substantially interfere with the activity of the compounds used in the methods of the present invention. The amount of carrier employed in conjunction with the compounds used in the methods of the present invention is sufficient to provide a practical quantity of material for administration per unit dose of the compounds. Techniques and compositions for making dosage forms useful in the methods of the present invention are described in the following references: Modern Pharmaceutics, Chapters 9 and 10, Banker & Rhodes, eds. (1979); Lieberman et al., Pharmaceutical Dosage Forms: Tablets (1981); and Ansel, Introduction to Pharmaceutical Dosage Forms, 2nd Ed., (1976).

Some examples of substances which can serve as carriers, or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol and polyethylene glycol; alginic acid; emulsifiers, such as the Tweens, e.g., Tween-20; wetting agents, such sodium lauryl sulfate; coloring agents; flavoring agents; tableting agents; stabilizers; antioxidants; preservatives; pyrogen-free water; isotonic saline; and phosphate buffer solutions. The choice of a carrier to be used in conjunction with the compounds useful in the methods of the present invention is typically determined by the way the compound is to be administered.

Preferably, the compounds useful in the methods of the present invention are administered topically. The carrier of the topical composition preferably aids penetration of the compounds as described herein into the skin to reach the environment of the hair follicle. Such topical compositions may be in any form including, for example, solutions, oils, creams, ointments, gels, lotions, pastes, shampoos, leave-on and rinse-out hair conditioners, milks, cleansers, moisturizers, sprays, aerosols and skin patches.

In topical compositions of the present invention, the compound as described herein may be in a form which would more readily penetrate into the skin and then be converted to the active form upon reaching the desired skin layer. Also, the topical compositions containing a compound as described herein can be admixed with a variety of carrier materials well known in the art, such as, for example, water, alcohols, aloe vera gel, allantoin, glycerine, vitamin A and E oils, mineral oil, propylene glycol and PPG-2 myristyl propionate.

Other materials suitable for use in topical carriers include, for example, emollients, solvents, humectants, thickeners and powders. Examples of each of these types of materials, which can be used singly or as mixtures of one or more materials, are as follows:

Emollients include, for example, stearyl alcohol, glyceryl monoricinoleate, glyceryl monostearate, propane-1,2-diol, butane-1,3-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polyethylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate and myristyl myristate. Propellants include, for example, propane, butane, isobutane, dimethyl ether, carbon dioxide and nitrous oxide. Solvents include, for example, ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide and tetrahydrofuran. Humectants include, for example, glycerin, sorbitol, sodium 2-pyrrolidone-5-carboxylate, soluble collagen, dibutyl phthalate and gelatin. Powders include, for example, chalk, talc, fullers earth, kaolin, starch, gums, colloidal silicon dioxide, sodium polyacrylate, tetraalkyl ammonium smectites, trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, and ethylene glycol monostearate.

The compounds used in the methods of the present invention may also be administered topically in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines. A preferred formulation for topical delivery of the compounds used in the methods of the present invention utilizes liposomes such as described in Dowton et al., "Influence of Liposomal Composition on Topical Delivery of Encapsulated Cyclosporin A: I. An *in vitro* Study Using Hairless Mouse Skin", *S.T.P. Pharma Sciences,* Vol. 3, pp. 404 - 407 (1993); Wallach and Philippot, "New Type of Lipid Vesicle: Novasome®", Liposome Technology, Vol. 1, pp. 141-156 (1993); U.S. Patent No. 4,911,928; and U.S. Patent No. 5,834,014.

The compounds of the present invention may also be topically administered by iontophoresis. See, e.g., www.unipr.it/arpa/dipfarm/erasmuslerasml4.html; Banga et al., "Hydrogel-based lontotherapeutic Delivery Devices for Transdermal Delivery of Peptide/Protein Drugs", *Pharm. Res.,* Vol. 10 (5), pp. 697-702 (1993); Ferry, "Theoretical Model of lontophoresis Utilized in Transdermal Drug Delivery", *Pharmaceutical Acta Helvetiae,* Vol. 70, pp. 279-287 (1995); Gangarosa et al., "Modem lontophoresis for Local Drug Delivery", *Int. J. Pharm,* Vol. 123, pp. 159-171 (1995); Green et al., "Iontophoretic Delivery of a Series of Tripeptides Across the Skin *in vitro", Pharm. Res.,* Vol 8, pp. 1121-1127 (1991); Jadoul et al., "Quantification and Localization of Fentanyl and TRH Delivered by lontophoresis in the Skin", *Int. J. Pharm.,* Vol. 120, pp. 22 I-8 (1995); O'Brien et al., "An Updated Review of its Antiviral Activity, Pharmacokinetic Properties and Therapeutic Efficacy", *Drugs,* Vol. 37, pp. 233-309 (1989); Parry et al., "Acyclovir Biovailability in Human Skin", *J. Invest. Dermatol.,* Vol. 98 (6), pp. 856-63 (1992); Santi et al., "Drug Reservoir Composition and Transport of Salmon Calcitonin in Transdermal lontophoresis", *Pharm. Res.,* Vol 14 (1), pp. 63-66 (1997); Santi et al., "Reverse lontophoresis - Parameters Determining Electroosmotic Flow: I. pH and Ionic Strength", *J. Control. Release,* Vol. 38, pp. 159-165 (1996); Santi et al., "Reverse lontophoresis - Parameters Determining Electroosmotic Flow: II. Electrode Chamber Formulation", *J. Control.Release,* Vol. 42, pp. 29-36 (1996); Rao et al., "Reverse lontophoresis: Noninvasive Glucose Monitoring *in vivo* in Humans", *Pharm. Res.,* Vol. 12 (12), pp. 1869-1873 (1995); Thvsman et al., "Human Calcitonin Delivery in Rats by lontophoresis", *J. Pharm. Pharmacol.,* Vol. 46, pp. 725-730 (1994); and Volpato et al., "Iontophoresis Enhances the Transport of Acyclovir through Nude Mouse Skin by Electrorepulsion and Electroosmosis", *Pharm. Res.,* Vol. 12 (11), pp.1623-1627 (1995).

Carriers for systemic administration include, for example, sugars, starches, cellulose and its derivatives, malt, gelatin, talc, calcium sulfate, vegetable oils, synthetic oils, polyols, alginic acid, phosphate buffer solutions, emulsifiers, isotonic saline and pyrogen-free water. Preferred carriers for parenteral administration include, for example, propylene glycol, ethyl oleate, pyrrolidone, ethanol and sesame oil. Preferably, the carrier in compositions for parenteral administration comprises at least about 90% by weight of the total composition.

Various oral dosage forms can be used, including such solid forms as tablets, capsules, granules and bulk powders. These oral forms comprise an effective amount, usually at least about 5%, and preferably from about 25% to about 50%, of a compound used in the methods of the present invention. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The carriers suitable for the preparation of unit dosage forms for oral administration are well known in the art. Tablets typically comprise conventional pharmaceutically compatible adjuvants as inert diluents, such as calcium carbonate, sodium carbonate, mannitol, lactose or cellulose; binders such as starch, gelatin or sucrose; disintegrants such as starch, alginic acid or croscarmelose; lubricants such as magnesium stearate, stearic acid or talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint or fruit flavors, are useful adjuvants for chewable tablets. Capsules (including time release and sustained release formulations) typically comprise one or more solid diluents as disclosed above. The selection of carrier components depends on secondary considerations like taste, cost and shelf stability, which are not critical for the methods *per se* of the present invention, and can readily be made by a person skilled in the art.

Orally administered compositions also include liquid solutions, emulsions, suspensions, powders, granules, elixirs, tinctures and syrups. The carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, Avicel RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents or colorants as described above.

Such compositions may also be coated by conventional methods, typically with pH or time-dependent coatings, such that the compound as described herein is released in the gastrointestinal tract in the desired vicinity or is released at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit coatings, waxes and shellac.

Other compositions useful for attaining systemic delivery of the compounds useful in the methods of the present invention include sublingual, buccal and nasal dosage forms. Such compositions typically comprise one or more soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents described above may also be included.

The above described compositions containing a compound as described herein may also optionally comprise an activity enhancer. The activity enhancer can be chosen from a wide variety of molecules which can function in different ways to enhance hair growth effects of a compound used in the methods of the present invention (see, for example, www.regrowth.com. for a listing of hair growth treatments). Particular classes of activity enhancers include hair growth stimulants and penetration enhancers.

Non-limiting examples of agents that stimulate hair growth and/or arrest hair loss which may additionally be used in the compositions described herein, including both systemic and topical compositions, include, for example, benzalkonium chloride, benzethonium chloride, phenol, estradiol, diphenhydramine hydrochloride, chlorpheniramine maleate, chlorophyllin derivatives, cholesterol, salicylic acid, cysteine, methionine, red pepper tincture, benzyl nicotinate, D,L-menthol, peppermint oil, calcium pantothenate, panthenol, castor oil, hinokitiol, prednisolone, resorcinol, monosaccharides and esterified monosaccharides, chemical activators of protein kinase C enzymes, glycosaminoglycan chain cellular uptake inhibitors, inhibitors of glycosidase activity, glycosaminoglycanase inhibitors, esters of pyroglutamic acid, hexosaccharic acids or acylated hexosaccharic acids, aryl-substituted ethylenes, N-acylated amino acids, cyclosporins, such as cyclosporin A, potassium channel blockers, such as minoxidil, 5-α-reductase inhibitors, such as finasteride, and androgen receptor antagonists, such as cyproterone acetate.

Preferred hair growth stimulants to be added to the compositions of the present invention are, for example, minoxidil and finasteride, with minoxidil being most preferred.

Non-limiting examples of penetration enhancers which may additionally be used in the compositions described herein include, for example, 2-methyl propan-2-ol, propan-2-ol, ethyl-2-hydroxypropanoate, hexan-2,5-diol, POE(2) ethyl ether, di(2-hydroxypropyl) ether, pentan-2,4-diol, acetone, POE(2) methyl ether, 2-hydroxypropionic acid, 2-hydroxyoctanoic acid, propan-1-ol, 1,4-dioxane, tetrahydrofuran, butan-1,4-diol, propylene glycol dipelargonate, polyoxypropylene 15 stearyl ether, octyl alcohol, POE ester of oleyl alcohol, oleyl alcohol, lauryl alcohol, dioctyl adipate, dicapryl adipate, di-isopropyl adipate, di-isopropyl sebacate, dibutyl sebacate, diethyl sebacate, dimethyl sebacate, dioctyl sebacate, dibutyl suberate, dioctyl azelate, dibenzyl sebacate, dibutyl phthalate, dibutyl azelate, ethyl myristate, dimethyl azelate, butyl myristate, dibutyl succinate, didecyl phthalate, decyl oleate, ethyl caproate, ethyl salicylate, iso-propyl palmitate, ethyl laurate, 2-ethyl-hexyl pelargonate, iso-propyl isostearate, butyl laurate, benzyl benzoate, butyl benzoate, hexyl laurate, ethyl caprate, ethyl caprylate, butyl stearate, benzyl salicylate, 2-hydroxypropanoic acid, 2-hydroxyoctanoic acid, dimethyl sulphoxide, N,N-dimethyl acetamide, N,N-dimethyl formamide, 2-pyrrolidone, 1-methyl-2-pyrrolidone, 5-methyl-2-pyrrolidone, 1,5-dimethyl-2-pyrrolidone, 1-ethyl-2-pyrrolidone, phosphine oxides, sugar esters, tetrahydrofurfural alcohol, urea, diethyl-m-toluamide and, 1-dodecylazacyloheptan-2-one.

In all of the foregoing, the compounds used in the methods of the present invention can be administered alone or as mixtures; and the compositions may further include additional drugs or excipients as appropriate for the indication, such as described above.

The present invention further relates to kits comprising a compound and/or composition as described herein and information and/or instructions by words, pictures, that use of the kit will provide treatment for hair loss in mammals (particularly humans) including, for example, arresting and/or reversing hair loss and/or promoting hair growth. In addition or in the alternative, the kit may comprise a compound and/or composition as described herein and information and/or instructions regarding methods of application of the compound and/or composition, preferably with the benefit of treating hair loss in mammals.

### EXAMPLES

In the examples below, "active ingredient" means a compound useful in the methods of the present invention, as described above.

### Example A

A composition for topical administration is made, comprising:

| COMPONENT | AMOUNT |
|---|---|
| Active ingredient | 1 % |
| Ethanol | 61 % |
| Propylene Glycol | 19 % |
| Dimethyl Isosorbide | 19 % |

### Example B

A composition for topical administration is made according to the method of Dowton et al, "Influence of Liposomal Composition on Topical Delivery of Encapsulated Cyclosporin A: I. An *in vitro* Study Using Hairless Mouse Skin", S.T.P. Pharma Sciences, Vol. 3, pp. 404 - 407 (1993), using an active ingredient in lieu of cyclosporin A and using the Novasome 1 for the non-ionic liposomal formulation.

A human male subject suffering from male pattern baldness is treated each day with the above composition. Specifically, for 6 weeks, the above composition is administered topically to the subject.

### Example C

Shampoos are made, comprising:

| COMPONENT | EG. C-1 | EG. C-2 | EG. C-3 | EG. C-4 |
|---|---|---|---|---|
| Ammonium Lauryl Sulfate | 11.5 % | 11.5 % | 9.5 % | 7.5 % |
| Ammonium Laureth Sulfate | 4 % | 3 % | 2 % | 2 % |
| Cocamide MEA | 2 % | 2 % | 2 % | 2 % |
| Ethylene Glycol Distearate | 2 % | 2 % | 2 % | 2 % |
| Cetyl Alcohol | 2 % | 2 % | 2 % | 2 % |
| Stearyl Alcohol | 1 .2 % | 1.2 % | 1.2 % | 1.2 % |
| Glycerin | 1% | 1% | 1% | 1% |
| Polyquaternium 10 | 0.5 % | 0.25 % | - | - |
| Polyquaternium 24 | - | - | 0.5 % | 0.25% |
| Sodium Chloride | 0.1% | 0.1% | 0.1% | 0.1 % |
| Sucrose Polyesters of Cottonate Fatty Acid | 3% | 3% | - | - |
| Sucrose Polyesters of Behenate Fatty Acid | 2 % | 3% | - | - |
| Polydimethyl Siloxane | - | - | 3 % | 2 % |
| Cocaminapropyl Betaine | - | 1 % | 3 % | 3% |
| Lauryl Dimethyl Amine Oxide | 1.5 % | 1.5 % | 1.5 % | 1.5% |
| Decyl Polyglucose | - | - | 1 % | 1 % |
| DMDM Hydantoin | 0.15 % | 0.15 % | 0.15 % | 0.15 % |
| Active ingredient | 0.2 % | 0.1% | 0.3% | 0.7% |
| Phenoxyethanol | 0.5% | 0.5% | 0.5% | 0.5% |
| Fragrance | 0.5% | 0.5% | 0.5% | 0.5% |
| Water | q.s. | q.s. | q.s. | q.s. |

A human subject suffering from male pattern baldness is treated by the methods of the present invention. Specifically, for 12 weeks, a shampoo above is used daily by the subject.

## Claims

1. Use of a compound of the formula 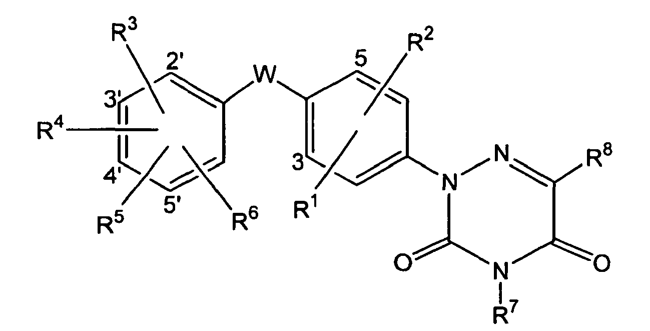 an isomer thereof, or a pharmaceutically acceptable salt of said compound or isomer;
wherein W is (a) -O-, (b) -S(O)ₘ-, (c) -NR³⁰-, (d) -C(O)-, (e) -HC=CH-, (f) -CH₂-, (g) -CHF-, (h) -CF₂- or (i) -CH(OH)-;
R¹ and R² are independently (a) hydrogen, (b) halogen, (c) -(C₁-C₆)alkyl, (d) -CN, (e) -OR¹² or (f) -trifluoromethyl;
R³ is (a) hydrogen, (b) halogen, (c) -(C₁-C₆)alkyl optionally substituted with one to three substituents independently selected from the group consisting of halogen, -OCF₃ and -CF₃, (d) -CN, (e) -OR¹², (f) -trifluoromethyl, (g) -NO₂, (h) -SO₂₋R¹³, (i) -C(O)₂R⁹, (j) -C(O)NR¹⁹R²⁰, (k) -C(O)R¹⁶, (l) -NR²¹C(O)-NR²¹R²², (m) -NR¹⁹⁻C(O)R²⁰ or (n) -NR¹⁷R¹⁸;
R⁴ is (a) -C(R¹⁴)(R¹⁵)(R¹⁶), (b) -(C₀-C₃)alkyl-NR¹⁷R¹⁸, (c) -C(O)NR¹⁹R²⁰, (d) -NR¹⁹-C(O)-R²⁰, (e) -(C₀-C₃)alkyl-NR²¹-C(O)-NR²¹R²², (f) -S(O)ₘ-R²², (g) -S(O)₂₋NR²¹R²², (h) -NR²¹-S(O)₂-R²², (i) -aryl, (j) -het, (k) -OR³³ or (l) halogen; provided that in substituents (f) and (h), R²² is other than -OR³⁴; and provided that when substituent (b) is -(C₀)alkyl-NR¹⁷R¹⁸, R¹⁸ is other than -C(O)-R²⁸ or -S(O)₂-R²⁹;
or R³ and R⁴ may be taken together to form a carbocyclic ring of Formula -(CH₂)_{b}- or a heterocyclic ring selected from the group consisting of -Q-(CH₂)_{c}- and -(CH₂)ⱼ-Q-(CH₂)ₖ- wherein Q is O, S or NR²⁵; wherein said carbocyclic ring is optionally substituted with one or more substituents independently selected from Group V; and wherein said heterocyclic ring is optionally substituted with one or more substituents independently selected from Group Z;
R⁵ is -OR²³;
or R⁴ and R⁵ may be taken together to form a heterocyclic ring selected from the group consisting of -CR³¹=CR³²-NH-, -N=CR³¹-NH-, -CR³¹=CR³²-O- and -CR³¹=CR³²-S-;
R⁶ is (a) hydrogen, (b) halogen, (c) -(C₁-C₆)alkyl optionally substituted with one to three substituents independently selected from the group consisting of halogen, -OCF₃ and -CF₃, (d) -CN, (e) -OR¹², (f) -trifluoromethyl, (g) -NO₂, (h) -SO₂-R¹³, (i) -C(O)₂R⁹, (j) -C(O)NR¹⁹R²⁰, (k) -C(O)R¹⁶, (I) -NR²¹C(O)NR²¹R²² (m) -NR¹⁹-C(O)R²⁰ or (n) -NR¹⁷R¹⁸;
R⁷ is (a) hydrogen, (b) -(C₁-C₄)alkyl wherein each carbon atom is optionally substituted with 1 to 3 halo atoms or (c) -(CH₂)ₙCOOR⁹;
R⁸ is (a) hydrogen, (b) -(C₁-C₆)alkyl, (c) -C(O)-OR⁹, (d) -C(O)NR¹⁰R¹¹ or (e) -CN; provided that in substituent (c), R⁹ is other than methyl or ethyl; and provided that in substitutent (d), R¹⁰ and R¹¹ are not both hydrogen;
R⁹ is (a) -(C₁-C₁₂)alkyl optionally substituted with one or more substitutents independently selected from Group V, (b) -(C₂-C₁₂)alkenyl optionally substituted with phenyl, (c) -(C₂-C₁₂)dialkenyl, (d) -(C₃-C₁₀)cycloalkyl, (e) -aryl or (f) -het;
R¹⁰ and R¹¹ are independently (a) hydrogen, (b) -(C₁-C₁₂)alkyl optionally substituted with one or more substituents independently selected from Group V, (c) -(C₃-C₁₀)cycloalkyl optionally substituted with one or more substituents independently selected from Group V, (d) -(C₂-C₁₂)alkenyl or (e) -het;
or R¹⁰ and R¹¹ for any occurrence may be taken together with the nitrogen atom to which are they attached to form het;
R¹² is (a) hydrogen or (b) -(C₁-C₆)alkyl wherein each carbon atom is optionally substituted with 1 to 3 fluoro atoms;
R¹³ is (a) -(C₁-C₁₂)alkyl optionally substituted with one or more substituents independently selected from Group V, (b) -(C₂-C₁₂)alkenyl, (c) -(C₃-C₁₀)cycloalkyl, (d) -NR¹⁷R¹⁸, (e) -aryl or (f) -het;
R¹⁴ is (a) hydrogen, (b) -(C₁-C₆)alkyl or (c)-O-R³⁴;
R¹⁵ is (a) hydrogen or (b) -(C₁-C₆)alkyl;
or R¹⁴ and R¹⁵ are taken together with the carbon atom to which they are attached to form a carbonyl group;
R¹⁶ is (a) hydrogen, (b) -(C₁-C₆)alkyl wherein each carbon atom is optionally substituted with 1 to 3 fluoro atoms, (c) -(C₀-C₆)alkyl-(C₃-C₁₀)cycloalkyl, (d) -(C₀₋C₆)alkyl-aryl or (e) -(C₀-C₆)alkyl-het;
R¹⁷ is (a) hydrogen, (b) -(C₁-C₁₂)alkyl optionally substituted with one or more substituents independently selected from Group V, (c) -aryl, (d) -het, (e) -OR³⁴ or (f) -(C₃-C₁₀)cycloalkyl;
R¹⁸ is (a) hydrogen, (b) -(C₁-C₁₂)alkyl optionally substituted with one or more substituents independently selected from Group V, (c) -aryl, (d) -het, (e) -C(O)-R²⁸, (f) -S(O)₂-R²⁹, (g) -OR³⁴ or (h) -(C₃-C₁₀)cycloalkyl;
or R¹⁷ and R¹⁶ for any occurrence are taken together with the nitrogen atom to which they are attached to form het;
R¹⁹ and R²⁰ for each occurrence are independently
(a) hydrogen, (b) -(C₁-C₁₂)alkyl optionally substituted with one or more substituents independently selected from Group V, (c) -(C₀-C₆)alkyl-aryl,
(d) -(C₀-C₆)alkyl-het, (e) -C(O)-NR²⁶R²⁷, (f) -C(O)-R²⁸, (g) -S(O)₂-R²⁹, (h) -OR³⁴ or (i) -(C₃-C₁₀)cycloalkyl;
or R¹⁹ and R²⁰ for any occurrence are taken together with the nitrogen atom to which they are attached to form het;
R²¹ and R²² for each occurrence are independently
(a) hydrogen, (b) -(C₁-C₁₂)alkyl optionally substituted with one to three substituents independently selected from Group V, (c)-aryl, (d) -het, (e) -(C₃-C₁₀)cycloalkyl or
(f) -OR³⁴;
or R²¹ and R²² are taken together with the nitrogen atom to which they are attached to form het;
R²³ is (a) hydrogen, (b) -(C₁-C₄)alkyl optionally substituted with one or more substituents independently selected from Group V or (c) -C(O)-R²⁴;
R²⁴ is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R²⁵ for each occurrence is independently (a) hydrogen, (b) -(C₁-C₆)alkyl,
(c) -COR²⁹ or (d) -SO₂R²⁹;
R²⁶ and R²⁷ for each occurrence are independently (a) hydrogen,
(b) -(C₁-C₆)alkyl, (c) -(C₃-C₁₀)cycloalkyl, (d) -(C₀-C₆)alkyl-aryl, or (e) -(C₀-C₆)alkyl-het,
R²⁸ is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R²⁹ is (a) -(C₁-C₁₂)alkyl optionally substituted with one or more substituents independently selected from Group V, (b) -(C₂-C₁₂)alkenyl,
(c) -(C₃-C₁₀)cycloalkyl, (d) -aryl or (e) -het;
R³⁰ is (a) hydrogen, (b) -(C₁-C₁₂)alkyl optionally substituted with one or more substituents independently selected from Group V, (c) -(C₁-C₁₂)alkenyl,
(d) -(C₃-C₁₀)cycloalkyl, (e) -C(O)-R³¹ or (f) -S(O)ₘ-R³²;
R³¹ is (a) hydrogen, (b) -(C₁-C₁₂)alkyl optionally substituted with one or more substituents independently selected from Group V, (c) -(C₂-C₁₂)alkenyl, (d) (C₃₋C₁₀)cycloalkyl, (e) -aryl, (f) -het or (g) -OR³⁴;
R³² is (a) hydrogen, (b) -(C₁-C₁₂)alkyl optionally substituted with one or more substituents independently selected from Group V, (c) -(C₂-C₁₂)alkenyl, (d) -(C₃₋C₁₀)cycloalkyl, (e) -aryl or (f) -het;
R³³ is (a) -(C₀-C₆)alkyl-aryl, (b) -(C₀-C₆)alkyl-het, (c) -(C₇-C₁₂)alkyl optionally substituted with one or more substituents independently selected from Group V, (d) -(C₁-C₆)alkyl wherein at least one carbon atom is substituted with 1 to 3 fluoro atoms, (e) -(C₂-C₁₂)alkenyl or (f) -(C₃-C₁₀)cycloalkyl;
R³⁴ is (a) -aryl, (b) -het , (c) -(C₁-C₁₂)alkyl optionally substituted with one or more substituents independently selected from Group V, (d) -(C₂-C₁₂)alkenyl or (e) -(C₃-C₁₀)cycloalkyl;
-(C₃-C₁₀)cycloalkyl for each occurrence is a fully or partially saturated mono-,bi- or tricyclic ring containing three to ten carbon atoms; wherein in the bicyclic ring, a monocyclic cycloalkyl ring is spiro fused to another cycloalkyl ring or is fused via two carbon atoms to a benzene ring or another cycloalkyl ring; and wherein in the tricyclic ring, a bicyclic ring is spiro fused to a cycloalkyl ring or is fused via two atoms to a benzene ring or another cycloalkyl ring;
said -(C₃-C₁₀)cycloalkyl optionally contains one to three bridging atoms independently selected from carbon, oxygen, sulfur and nitrogen; said bridging atoms are attached to two carbon atoms in the ring; and said bridging atoms are optionally substituted with one to three groups independently selected from -(C₁₋C₆)alkyl and hydroxy;
said cycloalkyl ring is optionally substituted on one ring if the moiety is monocyclic, on one or both rings if the moiety is bicyclic, or on one, two or three rings if the moiety is tricyclic, with one or more substitutents independently selected from Group V;
Group V is (a) -(C₁-C₆)alkyl optionally substituted with one or two hydroxy, (b) -(C₂-C₅)alkynyl, (c) -halogen, (d) -NR³⁵R³⁶, (e) -NO₂, (f) -OCF₃, (g) -OR³⁷, (h) -SR³⁷, (i) -oxo, (j) -trifluoromethyl, (k) -CN, (l) -C(O)NR³⁵-OH, (m) -COOR³⁵, (n) -O-C(O)-(C₁-C₆)alkyl, (o) -(C₃-C₁₀)cycloalkyl optionally substituted with CN, (p) -(C₀₋C₆)alkyl-aryl, (q) -(C₀-C₆)alkyl-het, (r) -C(O)-(C₁-C₆)alkyl or (s) -C(O)-aryl;
R³⁵ and R³⁶ for each occurrence are independently (a) hydrogen, (b) -(C₁₋C₆)alkyl or (c) -(C₀-C₆)alkyl-aryl;
R³⁷ is (a) hydrogen, (b) -(C₁-C₆)alkyl optionally substituted with one or more halo, hydroxy or methoxy, (c) -(C₀-C₆)alkyl-aryl or (d) -(C₀-C₆)alkyl-het;
aryl is (a) phenyl optionally substituted with one or more substituents independently selected from Group Z; (b) naphthyl optionally substituted with one or more substituents independently selected from Group Z or (c) biphenyl optionally substituted with one or more substituents independently selected from Group Z;
het for each occurrence is a 4-, 5-, 6-, 7- and 8-membered fully saturated, partially saturated or fully unsaturated mono-, bi- or tricyclic heterocyclic ring containing from one to four heteroatoms independently selected from the group consisting of oxygen, sulfur and nitrogen; wherein in the bicyclic ring, a monocyclic heterocyclic ring is spiro fused to a -(C₃-C₈)cycloalkyl ring or to another heterocyclic ring which is fully or partially saturated; or is fused via two atoms to a benzene ring, a -(C₃-C₈)cycloalkyl ring or another heterocyclic ring; and wherein in the tricyclic ring, a bicyclic ring is spiro fused to a -(C₃-C₈)cycloalkyl ring or to another heterocyclic ring which is fully or partially saturated; or is fused via two atoms to a benzene ring, a (C₃-C₆)cycloalkyl ring, or another heterocyclic ring;
said het optionally contains one to three bridging atoms independently selected from oxygen, sulfur and nitrogen; said bridging atoms are attached to two other atoms in the ring; and said bridging atoms are optionally substituted with one to three groups independently selected from -(C₁-C₆)alkyl and hydroxy;
said het optionally has one or two oxo groups substituted on carbon or one or two oxo groups substituted on sulfur;
said het is optionally substituted on carbon or nitrogen, on one ring if the moiety is monocyclic, on one or both rings if the moiety is bicyclic, or on one, two or three rings if the moiety is tricyclic, with one or more substituents independently selected from Group Z;
Group Z for each occurrence is independently (a) hydrogen, (b) halogen,
(c) trifluoromethyl, (d) hydroxy, (e) -OCF₃, (f) -CN, (g) -NO₂, (h) -(C₁-C₆)alkyl optionally substituted with one or more substituents independently selected from the group consisting of hydroxy, halogen, -OCF₃ and -CF₃, (i) -(C₂-C₆)alkenyl optionally substituted with phenyl, (j) -(C₂-C₅)alkynyl, (k) -(C₁-C₆)alkoxy, (l) -(C₀₋C₆)alkyl-phenyl optionally substituted with one or more substituents independently selected from the group consisting of halogen, -OCF₃, -CF₃, -(C₁-C₄)alkyl, -(C₁₋C₄)alkoxy and -C(O)CH₃, (m) -(C₀-C₆)alkyl-naphthyl optionally substituted with one or more substituents independently selected from the group consisting of halogen, -OCF₃, -CF₃, -(C₁-C₄)alkyl, -(C₁-C₄)alkoxy and -C(O)CH₃, (n) -C(O)₂R³⁵, (o) -(C₀₋C₆)alkyl-C(O)NR³⁵R³⁶, (p) -(C₀-C₆)alkyl-C(O)R³⁸, (q) -NR³⁵R³⁶, (r) -NR³⁵⁻C(O)NR³⁵R³⁶, (s) -NR³⁵-C(O)R³⁶, (t) -OR³⁷, (u) -SR³⁷, (v) -(C₃-C₁₀)cycloalkyl, (w) -(C₀-C₆)alkyl-pyridinyl optionally substituted with one or more -(C₁-C₆)alkyl which is optionally substituted with one or more substituents independently selected from the group consisting of hydroxy and halo, (x) -(C₀-C₆)alkyl-piperidinyl optionally substituted with one or more -(C₁-C₆)alkyl which is optionally substituted with one or more substituents independently selected from hydroxy and halo, (y) -SO₂-R³⁷, (z) -SO₂-NR³⁵R³⁶ or (a1) -S-phenyl-CH₂OH;
R³⁸ is (a) -(C₁-C₆)alkyl, (b) -(C₀-C₆)alkyl-phenyl, (c) -(C₀-C₆)alkyl-phenanthrenyl optionally substituted with one to three CF₃, (d) -(C₀-C₆)alkyl-pyrrolidinyl or (e) -(C₀-C₆)alkyl-morpholinyl;
or any two Z Groups for any occurrence in the same variable may be taken together to form (a) a carbocyclic ring of the formula -(CH₂)ₑ- or (b) a heterocyclic ring selected from the group consisting of -O(CH₂)_{f}O-, -(CH₂)₉NH- and -CH=CHNH-;
m is 0, 1 or 2;
n is 0, 1, 2 or 3;
b is 3, 4, 5, 6 or 7;
c, f, g, j and k are each independently 2, 3, 4, 5 or 6; and
e is 3, 4, 5, 6 or 7;
provided that in a compound of the above formula: 1) the substituent -C(R¹⁴)(R¹⁵)(R¹⁶) in R⁴ is other than (C₁-C₄)alkyl; and 2) R⁴ is halo only when R⁸ is -C(O)-OR⁹ or -C(O)NR¹⁰R¹¹;
for the manufacture of a medicament for the treatment of hair loss in a mammal.

2. Use according to claim 1 wherein the compound is selected from the group consisting of:
8-[[5-[2,6-dichloro-4-(4,5-dihydro-3,5-dioxo-1,2,4-triazine-2(3H)-yl)phenoxy]-2-hydroxyphenyl]sulfonyl]-spiro[8-azabicyclo[3.2.1]octane-3,2'-(3'H)-dihydro-furan];
2-{3,5-dichloro-4-[3-(3,3-dimethyl-piperidine-1-sulfonyl)-4-hydroxy-phenoxy]-phenyl}-2H-[1,2,4]triazine-3,5-dione;
2-{3,5-dichloro-4-[4-hydroxy-3-(3-methyl-3-phenyl-piperidine-1-sulfonyl)-phenoxy]-phenyl}-2H-[1,2,4]triazine-3,5-dione;
N-cyclohexyl-5-[2,6-dichloro-4-(3,5-dioxo-4,5-dihydro-3H-[1,2,4]triazin-2-yl)-phenoxy]-2-hydroxy-benzenesulfonamide;
N-bicyclo[2.2.1]hept-2-y1-5-[2,6-dichloro-4-(3,5-dioxo-4,5-dihydro-3H-[1,2,4]triazin-2-yl)-phenoxy]-2-hydroxy-benzamide;
2-{3,5-d ichloro-4-[3-(3,3-dimethyl-piperidine-1-carbonyl)-4-hydroxy-phenoxy]-phenyl}-2H-[1,2,4]triazine-3,5-dione;
N-bicyclo[2.2.1]hept-2-yl-5-[2,6-dichloro-4-(3,5-dioxo-4,5-dihydro-3H-[1,2,4]triazin-2-yl)-phenoxy]-2-hydroxy-benzamide;
2-{3,5-dichloro-4-[4-hydroxy-3-(3-methyl-3-phenyl-piperidine-1-carbonyl)-phenoxy]-phenyl}-2H-[1,2,4]triazine-3,5-dione;
5-[2,6-dichloro-4-(3,5-dioxo-4,5-dihydro-3H-[1,2,4]triazin-2-yl)-phenoxy]-N-(6,6-dimethyl-bicyclo[3.1.1]hept-2-yl)-2-hydroxy-benzamide;
2-{3,5-dichloro-4-[3-(3,5-dimethyl-piperidine-1-carbonyl)-4-hydroxy-phenoxy]-phenyl}-2H-[1,2,4]triazine-3,5-dione;
2-{3,5-dichloro-4-[4-hydroxy-3-(piperidine-1-carbonyl)-phenoxy]-phenyl}-2H-[1,2,4]triazine-3,5-dione;
N-cyclohexyl-5-[2,6-dichloro-4-(3,5-dioxo-4,5-dihydro-3H-[1,2,4]triazin-2-yl)-phenoxy]-2-hydroxy-benzamide;
2-{3,5-dichloro-4-[3-(3,4-dihydro-1H-isoquinoline-2-carbonyl)-4-hydroxy-phenoxy]-phenyl}-2H-[1,2,4]triazine-3,5-dione;
2-{4-[3-(4-fluoro-benzyl)-4-hydroxy-phenoxy]-3,5-dimethyl-phenyl}-2H-[1,2,4]triazine-3,5-dione; and
2-{3,5-dichloro-4-[3-(4-fluoro-benzoyl)-4-hydroxy-phenoxy]-phenyl}-2H-[1,2,4]triazine-3,5-dione.

3. Use according to claim 2 wherein the compound is:
2-(4-[3-(4-fluoro-benzyl)-4-hydroxy-phenoxy]-3,5-dimethyl-phenyl)-2H-[1,2,4]triazine-3,5-dione.

4. Use according to any one of claims 1 to 3 wherein the hair loss is selected from male pattern and female pattern baldness.

5. Use according to any one of claims 1 to 4 wherein the compound is cardiac-sparing.

6. Use according to any one of claims 1 to 4 wherein the mammal is a human being; and the medicament is for topical administration.

7. A topical pharmaceutical composition comprising a compound as defined in any one of claims 1 to 3 and further comprising a compound selected from finasteride, minoxidil and cyproterone acetate.

## Patentansprüche

1. Verwendung einer Verbindung der Formel 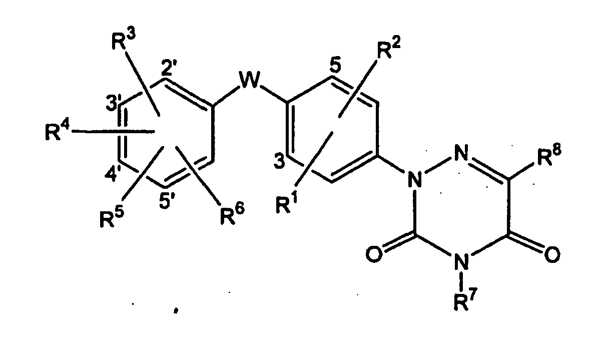 eines Isomers derselben oder eines pharmazeutisch akzeptablen Salzes der Verbindung oder des Isomers, worin W für (a) -O-, (b) -S(O)ₘ-, (c) -NR³⁰-, (d) -C(O)-, (e) -HC=CH-, (f) -CH₂-, (g) -CHF-, (h) -CF₂- oder (i) -CH(OH)- steht;
R¹ und R² unabhängig voneinander für (a) Wasserstoff, (b) Halogen, (c) -(C₁-C₆)Alkyl, (d) -CN, (e) -OR¹² oder (f) -Trifluormethyl stehen;
R³ für (a) Wasserstoff, (b) Halogen, (c) - (C₁-C₆)Alkyl, das optional mit einem bis drei Substituenten substituiert ist, die unabhängig voneinander aus der Gruppe von Halogen, -OCF₃ und -CF₃ ausgewählt sind, (d) -CN, (e) -OR¹², (f) -Trifluormethyl, (g) -NO₂, (h) -SO₂-R¹³, (i) -C(O)₂R⁹, (j) -C(O)NR¹⁹R²⁰, (k) -C(O)R¹⁶, (1) -NR²¹C(O)-NR²¹R²², (m) -NR¹⁹-C(O)R²⁰ oder (n) -NR¹⁷R¹⁸ steht;
R⁴ für (a) -C (R¹⁴) (R¹⁵) (R¹⁶), (b) - (C₀-C₃)Alkyl-NR¹⁷R¹⁸, (c) -C (O)NR¹⁹R²⁰, (d) -NR¹⁹-C(O)-R²⁰, (e) - (C₀-C₃)Alkyl-NR²¹⁻C(O)-NR²¹R²², (f) -S (O)ₘ-R²², (g) -S(O)₂-NR²¹R²², (h) -NR²¹⁻S (O)₂-R²², (i) -Aryl, (j) -Het, (k) -OR³³ oder (l) Halogen steht; mit der Maßgabe, dass in den Substituenten (f) und (h) R²² von -OR³⁴ verschieden ist, und mit der Maßgabe, dass, wenn der Substituent (b) -(C₀)Alkyl-NR¹⁷R¹⁸ ist, R¹⁸ von -C(O)-R²⁸ oder -S(O)₂-R²⁹ verschieden ist;
oder R³ und R⁴ zusammengenommen einen carbocyclischen Ring der Formel -(CH₂)_{b}- oder einen heterocyclischen Ring, der aus der Gruppe von -Q-(CH₂)_{c}- und -(CH₂)ⱼ-Q-(CH₂)ₖ-, worin Q O, S oder NR²⁵ ist, ausgewählt ist, bilden können, wobei der carbocyclische Ring optional mit einem oder mehreren Substituenten substituiert ist, die unabhängig voneinander aus der Gruppe V ausgewählt sind, und wobei der heterocyclische Ring optional mit einem oder mehreren Substituenten substituiert ist, die unabhängig voneinander aus der Gruppe Z ausgewählt sind;
R⁵ für -OR²³ steht;
oder R⁴ und R⁵ zusammengenommen einen heterocyclischen Ring bilden können, der aus der Gruppe von -CR³¹=CR³²-NH-, -N=CH³¹-NH-, -CR³¹=CR³²-O- und -CR³¹=CR³²-S- ausgewählt ist;
R⁶ für (a) Wasserstoff, (b) Halogen, (c) -(C₁-C₆)Alkyl, das optional mit einem bis drei Substituenten substituiert ist, die unabhängig voneinander aus der Gruppe von Halogen, -OCF₃ und -CF₃ ausgewählt sind, (d) -CN, (e) -OR¹², (f) -Trifluormethyl, (g) -NO₂, (h) -SO₂-R¹³, (i) -C(O)₂R⁹, (j) -C(O)NR¹⁹R²⁰, (k) -C(O)R¹⁶, (l) -NR²¹C(O)-NR²¹R²², (m) -NR¹⁹-C(O)R²⁰ oder (n) -NR¹⁷R¹⁸ steht;
R⁷ für (a) Wasserstoff, (b) -(C₁-C₄)Alkyl, worin jedes Kohlenstoffatom optional mit 1 bis 3 Halogenatomen substituiert ist, oder (c) -(CH₂)ₙCOOR⁹ steht;
R⁸ für (a) Wasserstoff, (b) -(C₁-C₆)Alkyl, (c) -C(O)-OR⁹, (d) -C(O)NR¹⁰R¹¹ oder (e) -CN steht, mit der Maßgabe, dass in dem Substituenten (c) R⁹ von Methyl oder Ethyl verschieden ist, und mit der Maßgabe, dass in dem Substituenten (d) R¹⁰ und R¹¹ nicht beide Wasserstoff sind; R⁹ für (a) -(C₁-C₁₂)Alkyl, das optional mit einem oder mehreren Substituenten substituiert ist, die unabhängig voneinander aus der Gruppe V ausgewählt sind, (b) -(C₂-C₁₂)-Alkenyl, das optional mit Phenyl substituiert ist, (c) -(C₂-C₁₂) Dialkenyl, (d) -(C₃-C₁₀)Cycloalkyl, (e) -Aryl oder (f) -Het steht;
R¹⁰ und R¹¹ unabhängig voneinander für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₃-C₁₀)Cycloalkyl, das optional mit einem oder mehreren Substituenten substituiert ist, die unabhängig voneinander aus der Gruppe V ausgewählt sind, (d) -(C₂-C₁₂)Alkenyl oder (e) -Het stehen;
oder R¹⁰ und R¹¹ bei jedem Vorkommen zusammen mit dem Stickstoffatom, an das sie gebunden sind, Het bilden können;
R¹² für (a) Wasserstoff oder (b) -(C₁₋₆)Alkyl, worin jedes Kohlenstoffatom optional mit 1 bis 3 Fluoratomen substituiert ist, steht;
R¹³ für (a) -(C₁-C₁₂)Alkyl, das optional mit einem oder mehreren Substituenten substituiert ist, die unabhängig voneinander aus der Gruppe V ausgewählt sind, (b) -(C₂-C₁₂)-Alkenyl, (c) -(C₃-C₁₀)Cycloalkyl, (d) -NR¹⁷R¹⁸, (e) -Aryl oder (f) -Het steht;
R¹⁴ für (a) Wasserstoff, (b) -(C₁-C₆)Alkyl oder (c) -O-R³⁴ steht;
R¹⁵ für (a) Wasserstoff oder (b) -(C₁-C₆)Alkyl steht;
oder R¹⁴ und R¹⁵ zusammengenommen mit dem Kohlenstoffatom, an das sie gebunden sind, eine Carbonylgruppe bilden;
R¹⁶ für (a) Wasserstoff, (b) -(C₁-C₆)Alkyl, worin jedes Kohlenstoffatom optional mit 1 bis 3 Fluoratomen substituiert ist, (c) -(C₀-C₆)Alkyl-(C₃-C₁₀) cycloalkyl, (d) - (C₀-C₆)Alkyl-aryl oder (e) -(C₀-C₆)Alkyl-het steht;
R¹⁷ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -Aryl, (d) -Het, (e) -OR³⁴ oder (f) -(C₃-C₁₀)Cycloalkyl steht;
R¹⁸ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -Aryl, (d) -Het, (e) -C(O)-R²⁸, (f) -S(O)₂-R²⁹, (g) -OR³⁴ oder (h) -(C₃-C₁₀)Cycloalkyl steht;
oder R¹⁷ und R¹⁸ bei jedem Vorkommen zusammengenommen mit dem Stickstoffatom, an das sie gebunden sind, Het bilden; R¹⁹ und R²⁰ bei jedem Vorkommen unabhängig voneinander für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₀-C₆)Alkyl-aryl, (d) - (C₀-C₆) Alkyl-het, (e) -C(O)-NR²⁶R²⁷, (f) -C(O)-R²⁸, (g) -S(O)₂-R²⁹, (h) -OR³⁴ oder (i) -(C₃-C₁₀) Cycloalkyl stehen;
oder R¹⁹ und R²⁰ bei jedem Vorkommen zusammengenommen mit dem Stickstoffatom, an das sie gebunden sind, Het bilden; R²¹ und R²² bei jedem Vorkommen unabhängig voneinander für (a) Wasserstoff, (b) -(C₁-C₁₂)Alkyl, das optional mit einem bis drei Substituenten substituiert ist, die unabhängig voneinander aus der Gruppe V ausgewählt sind, (c) -Aryl, (d) -Het, (e) -(C₃-C₁₀)Cycloalkyl oder (f) -OR³⁴ stehen;
oder R²¹ und R²² zusammengenommen mit dem Stickstoffatom, an das sie gebunden sind, Het bilden;
R²³ für (a) Wasserstoff, (b) -(C₁-C₄)Alkyl, das optional mit einem oder mehreren Substituenten substituiert ist, die unabhängig voneinander aus der Gruppe V ausgewählt sind, oder (c) -C(O)-R²⁴ steht;
R²⁴ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₂-C₁₂)Alkenyl, (d) -(C₃-C₁₀)Cycloalkyl, (e) -Aryl oder (f) -Het steht;
R²⁵ bei jedem Vorkommen unabhängig voneinander für (a) Wasserstoff, (b) -(C₁-C₆)Alkyl, (c) -COR²⁹ oder (d) -SO₂R²⁹ steht;
R²⁶ und R²⁷ bei jedem Vorkommen unabhängig voneinander für (a) Wasserstoff, (b)-(C₁-C₆)Alkyl, (c) -(C₃-C₁₀)Cycloalkyl, (d) -(C₀-C₆)Alkyl-aryl oder (e) -(C₀-C₆)Alkyl-het stehen;
R²⁸ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) - (C₂-C₁₂)Alkenyl, (d) -(C₃-C₁₀)Cycloalkyl, (e) -Aryl oder (f) -Het steht;
R²⁹ für (a) -(C₁-C₁₂)Alkyl, das optional mit einem oder mehreren Substituenten substituiert ist, die unabhängig voneinander aus der Gruppe V ausgewählt sind, (b) -(C₂-C₁₂)Alkenyl, (c) -(C₃-C₁₀)Cycloalkyl, (d) -Aryl oder (e) -Het steht;
R³⁰ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₁-C₁₂)Alkenyl, (d) -(C₃-C₁₀)Cycloalkyl, (e) -C(O)-R³¹ oder (f) -S(O)ₘ-R³² steht;
R³¹ für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₂-C₁₂)Alkenyl, (d) -(C₃-C₁₀)Cycloalkyl, (e) -Aryl, (f) -Het oder (g) -OR³⁴ steht;
R³² für (a) Wasserstoff, (b) -(C₁-C₁₂)Alkyl, das optional mit einem oder mehreren Substituenten substituiert ist, die unabhängig voneinander aus der Gruppe V ausgewählt sind, (c) -(C₂-C₁₂)Alkenyl, (d) -(C₃-C₁₀)Cycloalkyl, (e) -Aryl oder (f) -Het steht;
R³³ für (a) - (C₀-C₆)Alkyl-aryl, (b) -(C₀-C₆)Alkyl-het, (c) -(C₇-C₁₂)Alkyl, das optional mit einem oder mehreren Substituenten substituiert ist, die unabhängig voneinander aus der Gruppe V ausgewählt sind, (d) -(C₁-C₆)Alkyl, wobei mindestens ein Kohlenstoffatom mit 1 bis 3 Fluoratomen substituiert ist, (e) -(C₂-C₁₂)Alkenyl oder (f) -(C₃₋C₁₀) Cycloalkyl steht;
R³⁴ für (a) -Aryl, (b) -Het, (c) -(C₁-C₁₂)Alkyl, das optional mit einem oder mehreren Substituenten substituiert ist, die unabhängig voneinander aus der Gruppe V ausgewählt sind, (d)-(C₂-C₁₂)Alkenyl oder (e)-(C₃-C₁₀)Cycloalkyl steht;
-(C₃-C₁₀)Cycloalkyl bei jedem Vorkommen ein vollständig oder partiell gesättigter mono-, bi- oder tricyclischer Ring ist, der 3 bis 10 Kohlenstoffatome enthält, wobei in dem bicyclischen Ring ein monocyclischer Cycloalkylring in Spiroform an einen anderen Cycloalkylring kondensiert ist oder über zwei Kohlenstoffatome an einen Benzolring oder anderen Cycloalkylring kondensiert ist, und wobei in dem tricyclischen Ring ein bicyclischer Ring in Spiroform an einen Cycloalkylring kondensiert ist oder über zwei Atome an einen Benzolring oder einen anderen Cycloalkylring kondensiert ist;
das -(C₃-C₁₀)Cycloalkyl optional ein bis drei verbrückende Atome enthält, die unabhängig voneinander aus Kohlenstoff, Sauerstoff, Schwefel und Stickstoff ausgewählt sind, wobei die verbrückenden Atome an zwei Kohlenstoffatome in dem Ring gebunden sind und die verbrückenden Atome optional mit einer bis drei Gruppen substituiert sind, die unabhängig voneinander aus -(C₁-C₆)Alkyl und Hydroxy ausgewählt sind; der Cycloalkylring an einem Ring, wenn die Einheit monocyclisch ist, an einem oder beiden Ringen, wenn die Einheit bicyclisch ist, oder an einem, zwei oder drei Ringen, wenn die Einheit tricyclisch ist, optional mit einem oder mehreren Substituenten substituiert ist, die unabhängig voneinander aus der Gruppe V ausgewählt sind; die Gruppe V für (a) -(C₁-C₆)Alkyl, das optional mit einer oder zwei Hydroxygruppen substituiert ist, (b) -(C₂-C₅)-Alkinyl, (c) -Halogen, (d) -NR³⁵R³⁶, (e) -NO₂, (f) -OCF₃, (g) -OR³⁷, (h) -SR³⁷, (i) -Oxo, (j) -Trifluormethyl, (k) -CN, (l) -C(O)NR³⁵-OH, (m) -COOR³⁵, (n) -OC(O)-(C₁-C₆)-Alkyl, (O) -(C₃-C₁₀)Cycloalkyl, das optional mit CN substituiert ist, (p) - (C₀-C₆)Alkyl-aryl, (q) -(C₀-C₆)Alkyl-het, (r) -C(O)-(C₁-C₆)Alkyl oder (s) -C(O)-Aryl steht; R³⁵ und R³⁶ bei jedem Vorkommen unabhängig voneinander für (a) Wasserstoff, (b) -(C₁-C₆)Alkyl oder (c) -(C₀-C₆)Alkylaryl stehen;
R³⁷ für (a) Wasserstoff, (b) -(C₁-C₆)Alkyl, das optional mit einem oder mehreren Resten von Halogen, Hydroxy oder Methoxy substituiert ist, (c) -(C₀-C₆)Alkyl-aryl oder (d) -(C₀-C₆)Alkyl-het steht;
Aryl für (a) Phenyl, das optional mit einem oder mehreren Substituenten substituiert ist, die unabhängig voneinander aus der Gruppe Z ausgewählt sind, (b) Naphthyl, das optional mit einem oder mehreren Substituenten substituiert ist, die unabhängig voneinander aus der Gruppe Z ausgewählt sind, oder (c) Biphenyl, das optional mit einem oder mehreren Substituenten substituiert ist, die unabhängig voneinander aus der Gruppe Z ausgewählt sind, steht;
Het bei jedem Vorkommen für einen 4-, 5-, 6-, 7- und 8-gliedrigen vollständig gesättigten, partiell gesättigten oder vollständig ungesättigten mono-, bi- oder tricyclischen heterocyclischen Ring steht, der ein bis vier Heteroatome enthält, die unabhängig voneinander aus der Gruppe von Sauerstoff, Schwefel und Stickstoff ausgewählt sind; wobei in dem bicyclischen Ring ein monocyclischer heterocyclischer Ring in Spiroform an einen -(C₃-C₈)Cycloalkylring oder einen anderen heterocyclischen Ring, der vollständig oder partiell gesättigt ist, kondensiert ist oder über zwei Atome an einen Benzolring, einen -(C₃₋C₈)Cycloalkylring oder einen anderen heterocyclischen Ring kondensiert ist; und wobei in dem tricyclischen Ring ein bicyclischer Ring in Spiroform an einen -(C₃-C₈)Cycloalkylring oder einen anderen heterocyclischen Ring, der vollständig oder partiell gesättigt ist, kondensiert ist oder über zwei Atome an einen Benzolring, einen -(C₃₋C₈)Cycloalkylring oder einen anderen heterocyclischen Ring kondensiert ist;
wobei Het optional ein bis drei verbrückende Atome enthält, die unabhängig voneinander aus Sauerstoff, Schwefel und Stickstoff ausgewählt sind; wobei die verbrückenden Atome an zwei andere Atome in dem Ring gebunden sind und die verbrückenden Atome optional mit einer bis drei Gruppen substituiert sind, die unabhängig voneinander aus -(C₁-C₆)-Alkyl und Hydroxy ausgewählt sind;
das Het optional eine oder zwei, am Kohlenstoff substituierte Gruppen oder eine oder zwei, am Schwefel substituierte Gruppen aufweist;
das Het an einem Ring, wenn die Einheit monocyclisch ist, an einem oder beiden Ringen, wenn die Einheit bicyclisch ist, oder an einem, zwei oder drei Ringen, wenn die Einheit tricyclisch ist, am Kohlenstoff oder Stickstoff mit einem oder mehreren Substituenten, die unabhängig voneinander aus der Gruppe Z ausgewählt sind, optional substituiert ist; die Gruppe Z bei jedem Vorkommen unabhängig voneinander für (a) Wasserstoff, (b) Halogen, (c) Trifluormethyl, (d) Hydroxy, (e) -OCF₃, (f) -CN, (g) -NO₂, (h) -(C₁-C₆)-Alkyl, das optional mit einem oder mehreren Substituenten substituiert ist, die unabhängig voneinander aus der Gruppe von Hydroxy, Halogen, -OCF₃ und -CF₃ ausgewählt sind, (i) -(C₂-C₆)Alkenyl, das optional mit Phenyl substituiert ist, (j) -(C₂-C₅)Alkinyl, (k) -(C₁-C₆)Alkoxy, (l) -(C₀-C₆)-Alkyl-phenyl, das optional mit einem oder mehreren Substituenten substituiert ist, die unabhängig voneinander aus der Gruppe von Halogen, -OCF₃, -CF₃, -(C₁-C₄)Alkyl,-(C₁-C₄) Alkoxy und -C(O)CH₃ ausgewählt sind, (m) -(C₀₋C₆)Alkyl-naphthyl, das optional mit einem oder mehreren Substituenten substituiert ist, die unabhängig voneinander aus der Gruppe von Halogen, -OCF₃, -CF₃, -(C₁-C₄)Alkyl, -(C₁-C₄) Alkoxy und -C(O)CH₃ ausgewählt sind, (n) -C(O)₂R³⁵, (O) -(C₀-C₆)Alkyl-C(O)NR³⁵R³⁶, (p) -(C₀-C₆)Alkyl-C(O)R³⁸, (q) -NR³⁵R³⁶, (r) -NR³⁵-C(O)NR³⁵R³⁶, (s) -NR³⁵-C(O)R³⁶, (t) -OR³⁷, (u) -SR³⁷, (v) -(C₃-C₁₀)Cycloalkyl, (w) -(C₀-C₆)-Alkyl-pyridinyl, das optional mit einem oder mehreren -(C₁-C₆)Alkyl substituiert ist, das optional mit einem oder mehreren Substituenten substituiert ist, die unabhängig voneinander aus der Gruppe von Hydroxy und Halogen ausgewählt sind, (x) -(C₀-C₆)Alkyl-piperidinyl, das optional mit einem oder mehreren -(C₁-C₆)Alkyl substituiert ist, das optional mit einem oder mehreren Substituenten substituiert ist, die unabhängig voneinander aus der Gruppe von Hydroxy und Halogen ausgewählt sind, (y) -SO₂-R³⁷, (z)-SO₂-NR³⁵R³⁶ oder (a1) -S-Phenyl-CH₂OH steht; R³⁸ für (a) - (C₁-C₆)Alkyl, (b) - (C₀-C₆)Alkyl-phenyl, (c) -(C₀-C₆)Alkyl-phenanthrenyl, das optional mit einem bis drei CF₃ substituiert ist, (d) -(C₀-C₆)Alkyl-pyrrolidinyl oder (e) -(C₀-C₆)Alkyl-morpholinyl steht;
oder beliebige zwei Z-Gruppen bei jedem Vorkommen in der gleichen Variablen zusammengenommen werden können, wobei (a) ein carbocyclischer Ring der Formel -(CH₂)ₑ- oder (b) ein heterocyclischer Ring, der aus der Gruppe von -O(CH₂)_{f}O-, -(CH₂)_{g}NH- und -CH=CHNH- ausgewählt ist, gebildet wird;
m für 0, 1 oder 2 steht;
n für 0, 1, 2 oder 3 steht;
b für 3, 4, 5, 6 oder 7 steht;
c, f, g, j und k jeweils unabhängig voneinander für 2, 3, 4, 5 oder 6 stehen; und
e für 3, 4, 5, 6 oder 7 steht;
mit der Maßgabe, dass in einer Verbindung der obigen Formel
1) der Substituent -C(R¹⁴) (R¹⁵) (R¹⁶) in R⁴ von (C₁-C₄)Alkyl verschieden ist, und
2) R⁴ nur Halogen ist, wenn R⁸ für -C(O)-OR⁹ oder -C(O)NR¹⁰R¹¹ steht;
zur Herstellung eines Medikaments zur Behandlung von Haarausfall bei einem Säuger.

2. Verwendung nach Anspruch 1, wobei die Verbindung ausgewählt ist aus der Gruppe von:
8-[[5-[2,6-Dichlor-4-(4,5-dihydro-3,5-dioxo-1,2,4-triazin-2(3H)-yl)phenoxy]-2-hydroxyphenyl]sulfonyl]-spiro[8-azabicyclo[3.2.1]octan-3,2'-(3'H)-dihydro-furan];
2-{3,5-Dichlor-4-[3-(3,3-dimethyl-piperidin-1-sulfonyl)-4-hydroxy-phenoxy]-phenyl}-2H-[1,2,4]triazin-3,5-dion;
2-{3,5-Dichlor-4-[4-hydroxy-3-(3-methyl-3-phenyl-piperidin-1-sulfonyl)-phenoxy]-phenyl}-2H-[1,2,4]triazin-3,5-dion;
N-Cyclohexyl-5-[2,6-dichlor-4-(3,5-dioxo-4,5-dihydro-3H-[1,2,4]triazin-2-yl)-phenoxy]-2-hydroxy-benzolsulfonamid;
N-Bicyclo[2.2.1]hept-2-yl-5-[2,6-dichlor-4-(3,5-dioxo-4,5-dihydro-3H-[1,2,4]triazin-2-yl)-phenoxy]-2-hydroxy-benzamid;
2-{3,5-Dichlor-4-[3-(3,3-dimethyl-piperidin-1-carbonyl)-4-hydroxy-phenoxy]-phenyl}-2H-[1,2,4]triazin-3,5-dion;
N-Bicyclo[2.2.1]hept-2-yl-5-[2,6-dichlor-4-(3,5-dioxo-4,5-dihydro-3H-[1,2,4]triazin-2-yl)-phenoxy]-2-hydroxy-benzamid;
2-{3,5-Dichlor-4-[4-hydroxy-3-(3-methyl-3-phenyl-piperidin-1-carbonyl)-phenoxy]-phenyl}-2H-[1,2,4]triazin-3,5-dion;
5-[2,6-Dichlor-4-(3,5-dioxo-4,5-dihydro-3H-[1,2,4]triazin-2-yl)-phenoxy]-N-(6,6-dimethyl-bicyclo[3.1.1]hept-2-yl)-2-hydroxy-benzamid;
2-{3,5-Dichlor-4-[3-(3,5-dimethyl-piperidin-1-carbonyl)-4-hydroxy-phenoxy]-phenyl}-2H-[1,2,4]triazin-3,5-dion;
2-{3,5-Dichlor-4-[4-hydroxy-3-(piperidin-1-carbonyl)-phenoxy]-phenyl}-2H-[1,2,4]triazin-3,5-dion;
N-Cyclohexyl-5-[2,6-dichlor-4-(3,5-dioxo-4,5-dihydro-3H-[1,2,4]triazin-2-yl)-phenoxy]-2-hydroxy-benzamid;
2-{3,5-Dichlor-4-[3-(3,4-dihydro-1H-isochinolin-2-carbonyl)-4-hydroxy-phenoxy]-phenyl}-2H-[1,2,4]triazin-3,5-dion;
2-{4-[3-(4-Fluor-benzyl)-4-hydroxy-phenoxy]-3,5-dimethyl-phenyl}-2H-[1,2,4]triazin-3,5-dion und
2-{3,5-Dichlor-4-[3-(4-fluor-benzoyl)-4-hydroxy-phenoxy]-phenyl}-2H-[1,2,4]triazin-3,5-dion.

3. Verwendung nach Anspruch 2, wobei die Verbindung 2-{4-[3-(4-Fluor-benzyl)-4-hydroxy-phenoxy]-3,5-dimethyl-phenyl}-2H-[1,2,4]triazin-3,5-dion ist.

4. Verwendung nach einem der Ansprüche 1 bis 3, wobei der Haarausfall aus Alopecia androgenetica und dem weiblichen Typ der Alopecia androgenetica ausgewählt ist.

5. Verwendung nach einem der Ansprüche 1 bis 4, wobei die Verbindung herzschonend ist.

6. Verwendung nach einem der Ansprüche 1 bis 4, wobei der Säuger ein Mensch ist und das Medikament zur topischen Verabreichung dient.

7. Topische pharmazeutische Zusammensetzung, die eine Verbindung gemäß der Definition in einem der Ansprüche 1 bis 3 umfasst und ferner eine Verbindung umfasst, die aus Finasterid, Minoxidil und Cyproteronacetat ausgewählt ist.

## Revendications

1. Utilisation d'un composé de formule 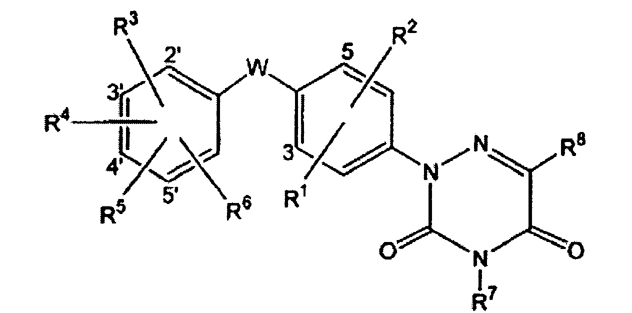
d'un de ses isomères, ou d'un sel pharmaceutiquement acceptable dudit composé ou isomère ;
formule dans laquelle W représente un groupe (a) -O-, (b) -S(O)ₘ-, (c) -NR³⁰-, (d) -C(O)-, (e) -HC=CH-, (f) -CH₂-, (g) -CHF, (h) -CF₂- ou (i) -CH(OH)- ;
R¹ et R² représentent indépendamment (a) un atome d'hydrogène, (b) un atome d'halogène, (c) un groupe -alkyle en C₁ à C₆, (d) un groupe -CN, (e) un groupe -OR¹² ou (f) un groupe trifluorométhyle ;
R³ représente (a) un atome d'hydrogène, (b) un atome d'halogène, (c) un groupe -alkyle en C₁ à C₆ facultativement substitué avec un à trois substituants choisis indépendamment dans le groupe consistant en des substituants halogéno, -OCF₃ et -CF₃, (d) un groupe -CN, (e) -OR¹², (f) un groupe trifluorométhyle, (g) -NO₂, (h) -SO₂-R¹³, (i) C(O)₂R⁹, (j) -C(O)NR¹⁹R²⁰, (k) -C(O)R¹⁶, (l) -NR²¹C(O)-NR²¹R²², (m) -NR¹⁹⁻C(O)R²⁰ ou (n) -NR¹⁷R¹⁸;
R⁴ représente un groupe (a) -C(R¹⁴)(R¹⁵)(R¹⁶), (b) - (alkyle en C₀ à C₃)-NR¹⁷R¹⁸, (c) -C(O)NR¹⁹R²⁰, (d)-NR¹⁹-C(O)-R²⁰, (e) - (alkyle en C₀ à C₃) -NR²¹-C(O)R²¹R²², (f) -S(O)ₘ-R²², (g) -S(O)₂₋NR²¹R²², (h) -NR²¹-S(O)₂-R²², (i) -aryle, (j) -het, (k) -OR³³ ou (l) halogéno ; sous réserve que, dans les substituants (f) et (h), R²² est autre qu'un groupe -OR³⁴ ; et sous réserve que, lorsque le substituant (b) est un substituant -(alkyle en C₀) -NR¹⁷R¹⁸, R¹⁸ est autre qu ' un groupe -C(O)-R²⁸ ou -S(O)₂-R²⁹ ;
ou bien R³ et R⁴ peuvent être pris conjointement pour former un noyau carbocyclique de formule -(CH₂)_{b}- ou un noyau hétérocyclique choisi dans le groupe consistant en des noyaux -Q-(CH₂)_{c}- et -(CH₂)ⱼ-Q-(CH₂)ₖ- dans lesquels Q représente O, S ou un groupe NR²⁵ ; ledit noyau carbocyclique étant facultativement substitué avec un ou plusieurs substituants choisis indépendamment dans le Groupe V ; et ledit noyau hétérocyclique étant facultativement substitué avec un ou plusieurs substituants choisis indépendamment dans le Groupe Z ;
R⁵ représente un groupe -OR²³;
ou bien R⁴ et R⁵ peuvent être pris conjointement pour former un noyau hétérocyclique choisi dans le groupe consistant en des groupes -CR³¹=CR³²-NH-, -N=CR³¹-NH-, -CR³¹=CR³²-O- et -CR³¹=CR³¹-S- ;
R⁶ représente (a) un atome d'hydrogène, (b) un atome d'halogène, (c) un groupe -alkyle en C₁ à C₆ facultativement substitué avec un à trois substituants choisis indépendamment dans le groupe consistant en des substituants halogéno, -OCF₃ et -CF₃, (d) un groupe -CN, (e) -OR¹², (f) un groupe trifluorométhyle, (g) -NO₂, (h) -SO₂-R¹³, (i) C(O)₂R⁹, (j) -C(O)NR¹⁹R²⁰, (k) -C(O)R¹⁶, (l) -NR²¹C(O)-NR²¹R²², (m) -NR¹⁹⁻C(O)R²⁰ ou (n) -NR¹⁷R¹⁸;
R⁷ représente (a) un atome d'hydrogène, (b) un groupe -alkyle en C₁ à C₄ dans lequel chaque atome de carbone est facultativement substitué avec 1 à 3 atomes d'halogène ou (c) un groupe -(CH₂)ₙCOOR⁹;
R⁸ représente (a) un atome d'hydrogène, (b) un groupe -alkyle en C₁ à C₆, (c) -C(O)-OR⁹, (d) -C(O)NR¹⁰R¹¹ ou (e) -CN ; sous réserve que dans le substituant (c), R⁹ est autre qu'un groupe méthyle et éthyle et sous réserve que, dans le substituant (d), R¹⁰ et R¹¹ ne représentent pas l'un et l'autre un atome d'hydrogène ;
R⁹ représente un groupe (a) -alkyle en C₁ à C₁₂ facultativement substitué avec un ou plusieurs substituants choisis indépendamment dans le Groupe V, (b) -alcényle en C₂ à C₁₂ facultativement substitué avec un substituant phényle, (c) -dialcényle en C₂ à C₁₂, (d) -cycloalkyle en C₃ à C₁₀, (e) -aryle ou (f) -het ;
R¹⁰ et R¹¹ représentent indépendamment (a) un atome d'hydrogène, (b) un groupe -alkyle en C₁ à C₁₂ facultativement substitué avec un ou plusieurs substituants choisis indépendamment dans le Groupe V, (c) -cycloalkyle en C₃ à C₁₀ facultativement substitué avec un ou plusieurs substituants choisis indépendamment dans le Groupe V, (d) -alcényle en C₂ à C₁₂ ou (e) -het ;
ou bien R¹⁰ et R¹¹, à n'importe quelle occurrence, peuvent être pris conjointement avec l'atome d'azote auquel ils sont fixés pour former un groupe het ;
R¹² représente (a) un atome d'hydrogène ou (b) un groupe -alkyle en C₁ à C₆ dans lequel chaque atome de carbone est facultativement substitué avec 1 à 3 atomes de fluor ;
R¹³ représente (a) un groupe -alkyle en C₁ à C₁₂ facultativement substitué avec un ou plusieurs substituants choisis indépendamment dans le Groupe V, (b) -alcényle en C₂ à C₁₂, (c) -cycloalkyle en C₃ à C₁₀, (d) -NR¹⁷R¹⁸, (e) -aryle ou (f) -het ;
R¹⁴ représente (a) un atome d'hydrogène, (b) un groupe -alkyle en C₁ à C₆ ou (c) -O-R³⁴ ;
R¹⁵ représente (a) un atome d'hydrogène ou (b) un groupe -alkyle en C₁ à C₆ ;
ou bien R¹⁴ et R¹⁵ sont pris conjointement avec l'atome de carbone auquel ils sont fixés pour former un groupe carbonyle ;
R¹⁶ représente (a) un atome d'hydrogène, (b) un groupe -alkyle en C₁ à C₆ dans lequel chaque atome de carbone est facultativement substitué avec 1 à 3 atomes de fluor, (c) -(alkyle en C₀ à C₆) - (cycloalkyle en C₃ à C₁₀), (d) - (alkyle en C₀ à C₆) -aryle ou (e) (alkyle en C₀ à C₆) -het ;
R¹⁷ représente (a) un atome d'hydrogène, (b) un groupe -alkyle en C₁ à C₁₂ facultativement substitué avec un ou plusieurs substituants choisis indépendamment dans le Groupe V, (c) -aryle, (d) -het, (e) -OR³⁴ ou (f) -cycloalkyle en C₃ à C₁₀ ;
R¹⁸ représente (a) un atome d'hydrogène, (b) un groupe -alkyle en C₁ à C₁₂ facultativement substitué avec un ou plusieurs substituants choisis indépendamment dans le Groupe V, (c) -aryle, (d) -het, (e)-C(O)-R²⁸, (f) -S(O)₂-R²⁹, (g) -OR³⁴ ou (h) -cycloalkyle en C₃ à C₁₀ ;
ou bien R¹⁷ et R¹⁸, à chaque occurrence, sont pris conjointement avec l'atome d'azote auquel ils sont fixés pour former un groupe het ;
R¹⁹ et R²⁰, à chaque occurrence, représentent indépendamment (a) un atome d'hydrogène, (b) un groupe -alkyle en C₁ à C₁₂ facultativement substitué avec un ou plusieurs substituants choisis indépendamment dans le Groupe V, (c) -(alkyle en C₀ à C₆) -aryle, (d) - (alkyle en C₀ à C₆) -het, (e) -C(O)-NR²⁶R²⁷, (f) -C(O)-R²⁸, (g) -S(O)₂-R²⁹, (h) -OR₃₄ ou (i) -cycloalkyle en C₃ à C₁₀ ;
ou bien R¹⁹ et R²⁰, à chaque occurrence, sont pris conjointement avec l'atome d'azote auquel ils sont fixés pour former un groupe het ;
R²¹ et R²², à chaque occurrence, représentent indépendamment (a) un atome d'hydrogène, (b) un groupe -alkyle en C₁ à C₁₂ facultativement substitué avec un à trois substituants choisis indépendamment dans le Groupe V, (c) -aryle, (d) -het, (e) -cycloalkyle en C₃ à C₁₀ ou (f) -OR³⁴ ;
ou bien R²¹ et R²² sont pris conjointement avec l'atome d'azote auquel ils sont fixés pour former un groupe het ;
R²³ représente (a) un atome d'hydrogène, (b) un groupe -alkyle en C₁ à C₄ facultativement substitué avec un ou plusieurs substituants choisis indépendamment dans le Groupe V ou (c) -C(O)-R²⁴ ;
R²⁴ représente (a) un atome d'hydrogène, (b) un groupe -alkyle en C₁ à C₁₂ facultativement substitué avec un ou plusieurs substituants choisis indépendamment dans le Groupe V, (c) -alcényle en C₂ à C₁₂, (d) -cycloalkyle en C₃ à C₁₀, (e) -aryle ou (f) -het ;
R²⁵, à chaque occurrence, représente indépendamment (a) un atome d'hydrogène, (b) un groupe -alkyle en C₁ à C₆, (c) -COR²⁹ ou (d) -SO₂R²⁹ ;
R²⁶ et R²⁷, à chaque occurrence, représentent indépendamment (a) un atome d'hydrogène, (b) un groupe -alkyle en C₁ à C₆, (c) -cycloalkyle en C₃ à C₁₀, (d) -(alkyle en C₀ à C₆) -aryle ou (e) - (alkyle en C₀ à C₆) - het ;
R²⁸ représente (a) un atome d'hydrogène, (b) un groupe -alkyle en C₁ à C₁₂ facultativement substitué avec un ou plusieurs substituants choisis indépendamment dans le Groupe V, (c) -alcényle en C₂ à C₁₂, (d) -cycloalkyle en C₃ à C₁₀, (e) -aryle ou (f) -het ;
R²⁹ représente (a) un groupe -alkyle en C₁ à C₁₂ facultativement substitué avec un ou plusieurs substituants choisis indépendamment dans le Groupe V, (b) -alcényle en C₂ à C₁₂, (c) -cycloalkyle en C₃ à C₁₀, (d) -aryle ou (e) -het ;
R³⁰ représente (a) un atome d'hydrogène, (b) un groupe -alkyle en C₁ à C₁₂ facultativement substitué avec un ou plusieurs substituants choisis indépendamment dans le Groupe V, (c) -alcényle en C₂ à C₁₂, (d) -cycloalkyle en C₃ à C₁₀, (e) -C(O)-R³¹ ou (f) -S(O)ₘ-R³² ;
R³¹ représente (a) un atome d'hydrogène, (b) un groupe -alkyle en C₁ à C₁₂ facultativement substitué avec un ou plusieurs substituants choisis indépendamment dans le Groupe V, (c) -alcényle en C₂ à C₁₂, (d) -cycloalkyle en C₃ à C₁₀, (e) -aryle, (f) -het ou (g) -OR³⁴ ;
R³² représente (a) un atome d'hydrogène, (b) un groupe -alkyle en C₁ à C₁₂ facultativement substitué avec un ou plusieurs substituants choisis indépendamment dans le Groupe V, (c) -alcényle en C₂ à C₁₂, (d) -cycloalkyle en C₃ à C₁₀, (e) -aryle ou (f) -het ;
R³³ représente (a) un groupe - (alkyle en C₀ à C₆) -aryle, (b) - (alkyle en C₀ à C₆) -het, (c) -alkyle en C₇ à C₁₂ facultativement substitué avec un ou plusieurs substituants choisis indépendamment dans le Groupe V, (d) -alkyle en C₁ à C₆ dans lequel au moins un atome de carbone est substitué avec 1 à 3 atomes de fluor, (e) -alcényle en C₂ à C₁₂ ou (f) -cycloalkyle en C₃ à C₁₀ ;
R³⁴ représente un groupe (a) -aryle, (b) -het, (c) -alkyle en C₁ à C₁₂ facultativement substitué avec un ou plusieurs substituants choisis indépendamment dans le Groupe V, (d) -alcényle en C₂ à C₁₂ ou (e) -cycloalkyle en C₃ à C₁₀ ;
le groupe -cycloalkyle en C₃ à C₁₀, à chaque occurrence, est un noyau mono-, bi- ou tricyclique, totalement ou partiellement insaturé, contenant trois à dix atomes de carbone ; dans lequel, dans le noyau bicyclique, un noyau cycloalkyle monocyclique est spirocondensé avec un autre noyau cycloalkyle ou est condensé par l'intermédiaire de deux atomes de carbone à un noyau benzénique ou un autre noyau cycloalkyle ; et dans lequel, dans le noyau tricyclique, un noyau bicyclique est spirocondensé avec un noyau cycloalkyle ou est condensé par l'intermédiaire de deux atomes à un noyau benzénique ou un autre noyau cycloalkyle ;
ledit groupe cycloalkyle en C₃ à C₁₀ contient facultativement un à trois atomes de pontage choisis indépendamment entre le carbone, l'oxygène, le soufre et l'azote ; lesdits atomes de pontage sont fixés à deux atomes de carbone dans le noyau ; et lesdits atomes de pontage sont facultativement substitués avec un à trois groupes choisis indépendamment entre des groupes -alkyle en C₁ à C₆ et hydroxy ;
ledit noyau cycloalkyle est facultativement substitué sur un noyau si le groupement est monocyclique, sur l'un des ou les deux noyaux si le groupement est bicyclique, ou bien sur un, deux ou trois noyaux si le groupement est tricyclique, avec un ou plusieurs substituants choisis indépendamment dans le Groupe V ;
le Groupe V est un groupe (a) -alkyle en C₁ à C₆ facultativement substitué avec un ou deux substituants hydroxy, (b) -alcényle en C₂ à C₅, (c) -halogéno, (d) -NR³⁵R³⁶, (e) -NO₂, (f) -OCF₃, (g) -OR³⁷, (h) -SR³⁷, (i) -oxo, (j) -trifluorométhyle, (k) -CN, (l) -C(O)NR³⁵-OH, (m) -COOR³⁵, (n) -O-C(O)-(alkyle en C₁ à C₆), (o) -cycloalkyle en C₃ à C₁₀ facultativement substitué avec un substituant CN, (p) -(alkyle en C₀ à C₆) -aryle, (q) - (alkyle en C₀ à C₆)-het, (r) -C(O)-(alkyle en C₁ à C₆) ou (s) -C-(O)-aryle ;
R³⁵ et R³⁶, à chaque occurrence, représentent indépendamment (a) un atome d'hydrogène, (b) un groupe -alkyle en C₁ à C₆ ou (c) (alkyle en C₀ à C₆)-aryle ;
R³⁷ représente (a) un atome d'hydrogène, (b) un groupe -alkyle en C₁ à C₆ facultativement substitué avec un ou plusieurs substituants halogéno, hydroxy ou méthoxy, (c) -(alkyle en C₀ à C₆) -aryle ou (d) - (alkyle en C₀ à C₆) -het ;
le groupe aryle est un groupe (a) phényle facultativement substitué avec un ou plusieurs substituants choisis indépendamment dans le Groupe Z ; (b) naphtyle facultativement substitué avec un ou plusieurs substituants choisis indépendamment dans le Groupe Z ; ou (c) biphényle facultativement substitué avec un ou plusieurs substituants choisis indépendamment dans le Groupe Z ;
het, à chaque occurrence, représente un noyau hétérocyclique mono-, di- ou tricyclique tétra-, penta-, hexa-, hepta- ou octogonal, totalement saturé, partiellement saturé ou totalement insaturé, contenant un à quatre hétéroatomes choisis indépendamment dans le Groupe consistant en l'oxygène, le soufre et l'azote ; dans lequel, dans le noyau bicyclique, un noyau hétérocyclique monocyclique est spirocondensé avec un noyau -cycloalkyle en C₃ à C₈ ou avec un autre noyau hétérocyclique qui est totalement ou partiellement saturé ; ou bien est condensé par l'intermédiaire de deux atomes avec un noyau benzénique, un noyau -cycloalkyle en C₃ à C₈ ou un autre noyau hétérocyclique ; et dans lequel, dans le noyau tricyclique, un noyau bicyclique est spiro condensé avec un noyau -cycloalkyle en C₃ à C₈ ou avec un autre noyau hétérocyclique qui est totalement ou partiellement saturé ; ou bien est condensé par l'intermédiaire de deux atomes avec un noyau benzénique, un noyau cycloalkyle en C₃ à C₆ ou un autre noyau hétérocyclique ;
ledit groupe het contient facultativement un à trois atomes de pontage choisis indépendamment entre l'oxygène, le soufre et l'azote ; lesdits atomes de pontage sont fixés à deux autres atomes dans le noyau ; et lesdits atomes de pontage sont facultativement substitués avec un à trois groupes choisis indépendamment entre des groupes -alkyle en C₁ à C₆ et hydroxy ;
ledit groupe het possède facultativement un ou deux groupes oxo présents comme substituants sur le carbone ou bien un ou deux groupes oxo présents comme substituants sur le soufre ;
ledit groupe het est facultativement substitué sur le carbone ou l'azote, sur un noyau si le groupement est monocyclique, sur l'un des ou les deux noyaux si le groupement est bicyclique, ou bien sur un, deux ou trois noyaux si le groupement est tricyclique, avec un ou plusieurs substituants choisis indépendamment dans le Groupe Z ;
le Groupe Z, à chaque occurrence, représente indépendamment (a) un atome d'hydrogène, (b) un atome d'halogène, (c) un groupe trifluorométhyle, (d) hydroxy, (e) -OCF₃, (f) -CN, (g) -NO₂, (h) -alkyle en C₁ à C₆ facultativement substitué avec un ou plusieurs substituants choisis indépendamment dans le groupe consistant en des substituants hydroxy, halogéno, -OCF₃ et -CF₃, (i) -alcényle en C₂ à C₆ facultativement substitué avec un substituant phényle, (j) -alcynyle en C₂ à C₅, (k) -alkoxy en C₁ à C₆, (l) -(alkyle en C₀ à C₆)-phényle facultativement substitué avec un ou plusieurs substituants choisis indépendamment dans le groupe consistant en des substituants halogéno, -OCF₃, -CF₃, -alkyle en C₁ à C₄, -alkoxy en C₁ à C₄ et -C(O)CH₃, (m) -(alkyle en C₀ à C₆)-naphtyle facultativement substitué avec un ou plusieurs substituants choisis indépendamment dans le groupe consistant en des substituants halogéno, -OCF₃, -CF₃, -alkyle en C₁ à C₄, -alkoxy en C₁ à C₄ et -C(O)CH₃, (n) -C(O)₂R³⁵, (o) (alkyle en C₀ à C₆) -C(O)NR³⁵R³⁶, (p) (alkyle en C₀ à C₆)-C(O)R³⁸, (q) -NR³⁵R³⁶, (r) -NR³⁵-C(O)NR³⁵R³⁶, (s) -NR³⁵⁻C(O)R³⁶, (t) -OR³⁷, (u) -SR³⁷, (v) -cycloalkyle en C₃ à C10, (w) -(alkyle en C₀ à C₆) -pyridinyle facultativement substitué avec un ou plusieurs substituants -alkyle en C₁ à C₆ qui sont facultativement substitués avec un ou plusieurs substituants choisis indépendamment dans le groupe consistant en des substituants hydroxy et halogéno, (x) -(alkyle en C₀ à C₆)-pipéridinyle facultativement substitué avec un ou plusieurs substituants -alkyle en C₁ à C₆ qui sont facultativement substitués avec un ou plusieurs substituants choisis indépendamment entre des substituants hydroxy et halogéno, (y) -SO₂-R³⁷, (z) -SO₂-NR³⁵R³⁶ ou (a1) -S-phényl-CH₂OH ;
R³⁸ représente un groupe (a) -alkyle en C₁ à C₆, (b) -(alkyle en C₀ à C₆) -phényle, (c) -(alkyle en C₀ à C₆) -phénanthrényle facultativement substitué avec un à trois substituants CF₃, (d) - (alkyle en C₀ à C₆) -pyrrolidinyle ou (e) (alkyle en C₀ à C₆)-morpholinyle ;
ou bien deux Groupes quelconques Z, à chaque occurrence, dans la même variable, peuvent être pris conjointement pour former (a) un noyau carbocyclique de formule -(CH₂)ₑ- ou (b) un noyau hétérocyclique choisi dans le groupe consistant en des noyaux -O(CH₂)_{f}O-, -(CH₂)_{g}NH- et -CH=CHNH- ;
m est égal à 0, 1 ou 2 ;
n est égal à 0, 1, 2 ou 3 ;
b est égal à 3, 4, 5, 6 ou 7 ;
c, f, g, j et k sont chacun égaux indépendamment à 2, 3, 4, 5 ou 6 ; et
e est égal à 3, 4, 5, 6 ou 7 ;
sous réserve que, dans un composé répondant à la formule précitée : 1) le substituant -C(R¹⁴) (R¹⁵) (R¹⁶) dans R⁴ est autre qu'un substituant alkyle en C₁ à C₄ ; et 2) R⁴ représente un groupe halogéno seulement lorsque R⁸ représente un groupe -C(O)-OR⁹ ou -C(O)NR¹⁰R¹¹ ;
pour la production d'un médicament destiné au traitement de la perte des cheveux chez un mammifère.

2. Utilisation suivant la revendication 1, dans laquelle le composé est choisi dans le groupe consistant en :
8-[[5-[2,6-dichloro-4-(4,5-dihydro-3,5-dioxo-1,2,4-triazine-2(3H)-yl)phénoxy]-2-hydroxyphényl]sulfonyl]-spiro-[8-azabicyclo[3.2.1]octane-3,2'-(3'H)-dihydro-furanne] ;
2-{3,5-dichloro-4-[3-(3,3-diméthyl-pipéridine-1-sulfonyl)-4-hydroxy-phénoxy]-phényl}-2H-[1,2,4]triazine-3,5-dione ;
2-{3,5-dichloro-4-[4-hydroxy-3-(3-méthyl-3-phényl-pipéridine-1-sulfonyl)-phénoxy]-phényl}-2H-[1,2,4]triazine-3,5-dione ;
N-cyclohexyl-5-[2,6-dichloro-4-(3,5-dioxo-4,5-dihydro-3H-[1,2,4]triazine-2-yl)-phénoxy]-2-hydroxy-benzènesulfonamide ;
N-bicyclo[2.2.1]hept-2-yl-5-[2,6-dichloro-4-(3,5-dioxo-4,5-dihydro-3H-[1,2,4]triazine-2-yl)-phénoxy]-2-hydroxy-benzamide ;
2-{3,5-dichloro-4-[3-(3,3-diméthyl-pipéridine-1-carbonyl)-4-hydroxy-phénoxy]-phényl}-2H-[1,2,4]triazine-3,5-dione ;
N-bicyclo[2.2.1]hept-2-yl-5-[2,6-dichloro-4-(3,5-dioxo-4,5-dihydro-3H-[1,2,4]triazine-2-yl)-phénoxy]-2-hydroxy-benzamide ;
2-{3,5-dichloro-4-[4-hydroxy-3-(3-méthyl-3-phényl-pipéridine-1-carbonyl)-phénoxy]-phényl}-2H-[1,2,4]triazine-3,5-dione ;
5-[2,6-dichloro-4-(3,5-dioxo-4,5-dihydro-3H-[1,2,4]triazine-2-yl)-phénoxy]-N-(6,6-diméthyl-bicyclo[3.1.1]hept-2-yl)-2-hydroxy-benzamide ;
2-{3,5-dichloro-4-[3-(3,5-diméthyl-pipéridine-1-carbonyl)-4-hydroxy-phénoxy]-phény}-2H-[1,2,4]triazine-3,5-dione ;
2-{3,5-dichloro-4-[4-hydroxy-3-(pipéridine-1-carbonyl)-phénoxy]-phényl}-2H-[1,2,4]triazine-3,5-dione ;
N-cyclohexyl-5-[2,6-dichloro-4-(3,5-dioxo-4,5-dihydro-3H-[1,2,4]triazine-2-yl)-phénoxy]-2-hydroxy-benzamide ;
2-{3,5-dichloro-4-[3-(3,4-dihydro-1H-isoguinoléine-2-carbonyl)-4-hydroxy-phénoxy]-phényl}-2H-[1,2,4]triazine-3,5-dione ;
2-{4-[3-(4-fluoro-benzyl)-4-hydroxy-phénoxy]-3,5-diméthyl-phényl}-2H-[1,2,4]triazine-3,5-dione ; et
2-{3,5-dichloro-4-[3-(4-fluoro-benzoyl)-4-hydroxy-phénoxy]-phényl}-2H-[1,2,4]triazine-3,5-dione.

3. Utilisation suivant la revendication 2, dans laquelle le composé est :
la 2-{4-[3-(4-fluoro-benzyl)-4-hydroxy-phénoxy]-3,5-diméthyl-phényl}-2H-[1,2,4]triazine-3,5-dione.

4. Utilisation suivant l'une quelconque des revendications 1 à 3, dans laquelle la perte des cheveux est choisie entre la calvitie hippocratique masculine et la calvitie hippocratique féminine.,

5. Utilisation suivant l'une quelconque des revendications 1 à 4, dans laquelle le composé a une action d'épargne cardiaque.

6. Utilisation suivant l'une quelconque des revendications 1 à 4, dans laquelle le mammifère est un être humain, et le médicament est destiné à l'administration topique.

7. Composition pharmaceutique topique comprenant un composé tel que défini dans l'une quelconque des revendications 1 à 3 et comprenant en outre un composé choisi entre le finastéride, le minoxidil et l'acétate de cyprotérone.
